# EUROPEAN PATENT SPECIFICATION

(11) **EP 1 318 835 B1**
(45) Date of publication and mention of the grant of the patent: **16.07.2008**
(21) Application number: 01967535.4
(22) Date of filing: 20.09.2001
(51) Int. Cl.: A61K 39/39, A61K 48/00, A61P 31/00

(54) **USE OF IMIDAZOQUINOLINAMINES AS ADJUVANTS IN DNA VACCINATION**
VERWENDUNG VON IMIDAZOCHINOLINAMINEN ALS ADJUVANTIEN IN DNA-IMPFUNG
UTILISATION D'IMIDAZOQUINOLINAMINES COMME ADJUVANTS DANS UNE VACCINATION PAR L'ADN

(30) Priority: 20.09.2000 GB 0023008
(43) Date of publication of application: 18.06.2003
(73) Proprietor: GLAXO GROUP LIMITED, Greenford, Middlesex UB6 ONN (GB)
(72) Inventor: THOMSEN, Lindy, Louise GlaxoSmithKline, Stevenage Hertfordshire SG1 2NY (GB); TITE, John, Philip GlaxoSmithKline, Stevenage Hertfordshire SG1 2NY (GB); TOPLEY, Peter GlaxoSmithKline, Stevenage Hertfordshire SG1 2NY (GB)
(74) Representative: Easeman, Richard Lewis
(86) International application number: PCT/GB2001/004207
(87) International publication number: WO 2002/024225

(56) References cited:
- WO-A-93/20847
- WO-A1-00/12121
- VASILAKOS J P ET AL: "Adjuvant activities of immune response modifier R - 848 comparison with CpG ODN." CELLULAR IMMUNOLOGY, (2000 AUG 25) 204 (1) 64-74. , XP001037913
- SAUDER D N: "Immunomodulatory and pharmacologic properties of imiquimod." JOURNAL OF THE AMERICAN ACADEMY OF DERMATOLOGY, (2000 JUL) 43 (1 PT 2) S6-11. REF: 29 , XP001037946
- HARRISON ET AL: 'Effect of S28463 as an adjuvant for an immunotherpeutic HSV glycoprotein D (gD) DNA vaccine: Reduction of recurrent genital HSV disease in guinea pigs' ICAAC 36TH page H121, XPSER: ICAAC 36TH
- TOMAI ET AL: 'Immunomodulating and antiviral activities of the imidazoquinoline S-28463' ANTIVIRAL RESEARCH vol. 28, 1995, pages 253 - 264
- SASAKI ET AL: 'Monophosphoryl Lipid A enhances both humoral and cell-mediated immune responses to DNA vaccination against human immunodeficiency virus type 1' INFECTIOIN AND IMMUNITY vol. 65, no. 9, September 1997, pages 3520 - 3528
- DR SMITH ET AL: "Oxford Dictionary of Biochemistry and Molecular Biology" 1997, OXFORD UNITED PRESS , NEW YORK * page 18 *
- XIANG; ERTL: 'Manipulation of the immune response to a plasmid-encoded viral antigen by coinoculation of plasmids expressing cytokines' IMMUNITY vol. 2, 1995, pages 129 - 135
- HARMS ET AL: 'Regulation of transgene expression in genetic immunization' BRAZILIAN JOURNAL OF MEDICAL AND BIOLOGICAL RESEARCH vol. 32, 1999, pages 155 - 162

## Description

### Field of the Invention

The present invention relates to improvements in DNA vaccination and in particular, but not exclusively, to vaccine compositions, methods of vaccinating a mammal against disease states, and to the use of certain compounds in the manufacture of medicaments.

### Background of the Invention

Traditional vaccination techniques which involve the introduction into an animal system of an antigen which can induce an immune response in the animal, and thereby protect the animal against infection, have been known for many years. Following the observation in the early 1990's that plasmid DNA could directly transfect animal cells *in vivo,* significant research efforts have been undertaken to develop vaccination techniques based upon the use of DNA plasmids to induce immune responses, by direct introduction into animals of DNA which encodes for antigenic peptides. Such techniques, which are referred to as "DNA immunisation" or "DNA vaccination" have now been used to elicit protective antibody (humoral) and cell-mediated (cellular) immune responses in a wide variety of pre-clinical models for viral, bacterial and parasitic diseases. Research is also underway in relation to the use of DNA vaccination techniques in treatment and protection against cancer, allergies and autoimmune diseases.

DNA vaccines usually consist of a bacterial plasmid vector into which is inserted a strong promoter, the gene of interest which encodes for an antigenic peptide and a polyadenylation/transcriptional termination sequence. The immunogen which the gene of interest encodes may be a full protein or simply an antigenic peptide sequence relating to the pathogen, tumour or other agent which is intended to be protected against. The plasmid can be grown in bacteria, such as for example *E. coli* and then isolated and prepared in an appropriate medium, depending upon the intended route of administration, before being administered to the host.

Helpful background information in relation to DNA vaccination is provided in "Donnelly, J et al Annual Rev. Immunol. (1997) 15:617-648, the disclosure of which is included herein in its entirety by way of reference.

There are a number of advantages of DNA vaccination relative to traditional vaccination techniques. First, it is predicted that because the proteins which are encoded by the DNA sequence are synthesised in the host, the structure or conformation of the protein will be similar to the native protein associated with the disease state. It is also likely that DNA vaccination will offer protection against different strains of a virus, by generating cytotoxic T lymphocyte responses that recognise epitopes from conserved proteins. Furthermore, because the plasmids are introduced directly to host cells where antigenic protein can be produced, a long-lasting immune response will be elicited. The technology also offers the possibility of combining diverse immunogens into a single preparation to facilitate simultaneous immunisation in relation to a number of disease states.

Despite the numerous advantages associated with DNA vaccination relative to traditional vaccination therapies, there is nonetheless a desire to develop adjuvant compounds which will serve to increase the immune response induced by the protein which is encoded by the plasmid DNA administered to an animal.

DNA vaccination is sometimes associated with an inappropriate deviation of immune response from a Th1 to a Th2 response, especially when the DNA is administered directly to the epidermis (Fuller and Haynes Hum. Retrovir. (1994) 10:1433-41). It is recognised that the immune profile desired from a nucleic acid vaccine depends on the disease being targeted. The preferential stimulation of a Th1 response is likely to provide efficacy of vaccines for many viral diseases and cancers, and a dominant Th2 type of response may be effective in limiting allergy and inflammation associated with some autoimmune diseases. Accordingly, ways to quantitatively raise the immune response or to shift the type of response to that which would be most efficacious for the disease indication, may be useful.

Accordingly, it is one object of the present invention to provide adjuvant compounds which can be used in conjunction with DNA vaccination procedures. It is also an object to provide compositions including the adjuvants concerned, as well as methods of improved DNA vaccination involving such adjuvants. Other objects of the present invention will become apparent from the following detailed description thereof. To date, however, meeting these objects has proven difficult, largely due to mechanistic differences associated with DNA vaccination, as compared with traditional vaccine techniques. Also, identification of suitable compounds is not straightforward, a number of known immunopotentiating agents have been tried in combination with DNA vaccination techniques with limited, or at best mixed success. For example, coadministration of the genes for IFN-γ and rabies virus glycoprotein had an inhibitory effect on both T helper cell response and the antibody response (Xiang and Ertl).

In an abstract (36^{th} JCAAC, H121) Harrison et al show that a DNA vaccine against HSV and the imidazoquinoline derivative S28463 have an additive therapeutic effect, when administered 7 days apart.

With this background in mind, it is most surprising to note that the present inventors report adjuvant compounds that are effective in promoting an improved immune response, in particular an improved cellular immune response when used as adjuvants in DNA vaccination. Imidazoquinolineamine derivatives are inducers of cytokines, including IFN-α, IL-6 and TNF-α (See, e.g. Reiter et al, J. Leukocyte Biology (1994) 55:234-240). These compounds and processes for their preparation have been disclosed in PCT patent application publication number WO 94/17043. The present inventors have shown that these derivatives may be effectively used as adjuvants in DNA vaccination.

### Summary of the Invention

According to one embodiment of the present invention there is provided a vaccine comprising (i) an adjuvant component comprising a 1 H-imidazo[4,5-c]quinolin-4-amine derivative and (ii) an immunogen component comprising a nucleotide sequence encoding an antigenic peptide or protein associated with a disease state, wherein the adjuvant component enhances the immune responses in a mammal to the antigenic peptide or protein and wherein the adjuvant component is administered between about 1 day prior to and about 3 days post administration of the immunogen component.

In a further embodiment there is provide the use of imidazo [4,5-c]quinolin-1H-imidazo[4,5-c]quinolin-4-amine derivative in the manufacture of a medicament for enhancing immune responses initiated by an antigenic peptide in the treatment of a mammal in a disease state selected from viral, bacterial or parasitic infection, cancer, allergy or autoimmune disorder, said peptide being expressed as a result of administration to a mammal of a nucleotide sequence encoding for said peptide, and wherein the 1H-imidazo[4,5-c]quinolin-4-amine derivative is administered between about 1 day prior to and about 3 days post administration of the nucleotide sequence encoding for said peptide.

Preferably the 1H-imidazo[4,5-c]quinolin-4-amine-derivative is a compound defined by one of formulae I-VI defined herein. More preferably, it is a compound defined by formula VI. Particularly preferred is when the 1 H-imidazo[4,5-c]quinolin-4-amine derivative is a compound of formula VI selected from the group consisting of
1-(2-methylpropyl)-1H-imidazo[4,5-c]quinolin-4-amine;
1-(2-hydroxy-2-methylpropyl)-2-methyl-1H-imidazo[4,5-c]quinolin-4-amine;
1-(2,hydroxy-2-methylpropyl)-1H-imidazo[4,5-c]quinolin-4-amine;
1-(2-hydroxy-2-methylpropyl)-2-ethoxymethyl-1-H-imidazo[4,5-c]quinolin-4-amine

### Brief Description of the Figures

**Figure 1****.**
   Imiquimod increases the cytotoxic T-cell response following vaccination with a plasmid encoding nucleoprotein from influenza virus (pVAC1.PR). pVAC1 is the vector control.
**Figure 2****.**
   Imiquimod increases the clonal expansion of CD4 T cells in vivo following vaccination with a plasmid encoding ovalbumin protein, as measured by increased proliferation of CD4+ T cells.
**Figure 3****.**
   Imiquimod increases the number of activated CD4 T cells *in vivo* following vaccination with a plasmid encoding ovalbumin protein, as measured by increased IFN-γ and IL-4 producing cells.
**Figure 4****.**
   Imiquimod induces both Th1 and Th2 responses *in vivo* following vaccination with a plasmid encoding ovalbumin protein, as measured by increased IFN-γ and IL-4 producing cells, respectively.
**Figure 5****.**
   Imiquimod increases the cytotoxic T-cell response following vaccination with a plasmid encoding the HIV antigens Gag and Nef (WRG7077.Gag/Nef). WRG7077 is the vector control.
**Figure 6****.**
   Resiquimod (1-(2-hydroxy-2-methylpropyl)-2-ethoxymethyl-1-H-imidazo[4,5-c]quinolin-4-amine), an analogue of imiqumod, increases the number of activated CD4 T cells *in vivo* following vaccination with a plasmid encoding ovalbumin protein, as measured by increased IFN-γ and IL-4 producing cells (6A and 6B, respectively).
**Figure 7****.**
   Two analogues of imiquimod, 1-(2-hydroxy-2-methylpropyl)-2-methyl-1H-imidazo[4,5-c]quinolin-4-amine and 1-(2,hydroxy-2-methylpropyl)-1H-imidazo[4,5-c]quinolin-4-amine, increase the number of activated CD4 T cells *in vivo* following vaccination with a plasmid encoding ovalbumin protein, as measured by increased IFN-γ and IL-4 producing cells (7A and 7B, respectively).
**Figure 8****.**
   Topical application of imiquimod increases the cytotoxic T-cell response following vaccination with a plasmid encoding the HIV antigens Gag and Nef (WRG7077.Gag/Nef). WRG7077 is the vector control.
**Figure 9****.**
   Imiquimod delays the growth of tumours in animals challenged with ovalbumin-expressing EG7.OVA tumour cells after immunisation with the plasmid encoding ovalbumin.
**Figure 10****.**
   Imiquimod reduces the tumourigenicity of ovalbumin-expressing EG7.OVA tumour cells implanted into animals pre-immunised with the plasmid encoding ovalbumin.

### Detailed Description of the Invention

Throughout this specification and the appended claims, unless the context requires otherwise, the words "comprise" and "include" or variations such as "comprising", "comprises", "including", "includes", etc., are to be construed inclusively, that is, use of these words will imply the possible inclusion of integers or elements not specifically recited.

As described above, the present invention relates to immunogenic compositions such as vaccine compositions, vaccination methods, and to improvements of methods of vaccination involving the introduction into a mammal of nucleotide sequence which encodes for an immunogen which is an antigenic protein or peptide, such that the protein or peptide will be expressed within the mammalian body to thereby induce an immune response within the mammal against the antigenic protein or peptide. Such methods of vaccination are well known and are fully described in Donnelly *et al* as referred to above.

As used herein the term vaccine composition refers to a combination of a immunogen component comprising a nucleotide sequence encoding an immunogen, and an adjuvant component comprising a 1H-imidazo [4,5-c] quinolin-4-amine derivative. The combination is, for example, in the form of an admixture of the two components in a single pharmaceutically acceptable formulation or in the form of separate, individual components, for example in the form of a kit comprising an immunogen component comprising the nucleotide sequence encoding an immunogen, and an adjuvant component comprising the 1H-imidazo[4,5-c]quinolin-4-amine, wherein the two components are for separate, sequential or simultaneous administration. Preferably, the administration of the two components is substantially simultaneous.

The IH-imidazo[4,5-c]quinolin-4-amine derivative as referred to throughout the specification and the claims is preferably a compound defined by one of Formulas I-VI below: 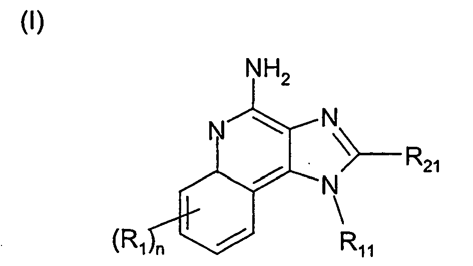 wherein
R₁₁ is selected from the group consisting of straight or branched chain alkyl, hydroxyalkyl, acyloxyalkyl, benzyl, (phenyl)ethyl and phenyl, said benzyl, (phenyl)ethyl or phenyl substituent being optionally substituted on the benzene ring by one or two moieties independently selected from the group consisting of alkyl of one to about four carbon atoms, alkoxy of one to about four carbon atoms and halogen, with the proviso that if said benzene ring is substituted by two of said moieties, then said moieties together contain no more than 6 carbon atoms; R₂₁ is selected from the group consisting of hydrogen, alkyl of one to about eight carbon atoms, benzyl, (phenyl)ethyl and phenyl, the benzyl, (phenyl)ethyl or phenyl substituent being optionally substituted on the benzene ring by one or two moieties independently selected from the group consisting of alkyl of one to about four carbon atoms, alkoxy of one to about four carbon atoms and halogen, with the proviso that when the benzene ring is substituted by two of said moieties, then the moieties together contain no more than 6 carbon atoms; and each R₁ is independently selected from the group consisting of hydrogen, alkoxy of one to about four carbon atoms, halogen and alkyl of one to about four carbon atoms, and n is an integer from 0 to 2, with the proviso that if n is 2, then said R₁₁ groups together contain no more than 6 carbon atoms; 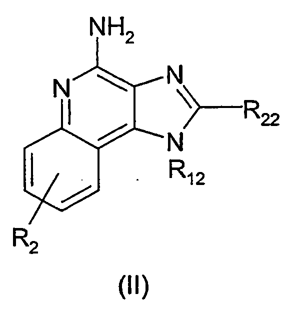 wherein
R₁₂ is selected from the group consisting of straight chain or branched chain alkenyl containing 2 to about 10 carbon atoms and substituted straight chain or branched chain alkenyl containing 2 to about 10 carbon atoms, wherein the substituent is selected from the group consisting of straight chain or branched chain alkyl containing 1 to about 4 carbon atoms and cycloalkyl containing 3 to about 6 carbon atoms; and cycloalkyl containing 3 to about 6 carbon atoms substituted by straight chain or branched chain alkyl containing 1 to about 4 carbon atoms; and R₂₂ is selected from the group consisting of hydrogen, straight chain or branched chain alkyl containing one to about eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl containing one to about four carbon atoms, straight chain or branched chain alkoxy containing one to about four carbon atoms, and halogen, with the proviso that when the benzene ring is substituted by two such moieties, then the moieties together contain no more than 6 carbon atoms; and each R₂ is independently selected from the group consisting of straight chain or branched chain alkoxy containing one to about four carbon atoms, halogen, and straight chain or branched chain alkyl containing one to about four carbon atoms, and n is an integer from zero to 2, with the proviso that if n is 2, then said R₂ groups together contain no more than 6 carbon atoms; 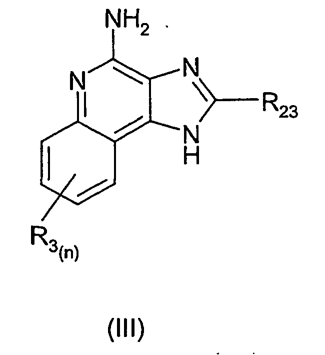 wherein
R₂₃ is selected from the group consisting of hydrogen, straight chain or branched chain alkyl of one to about eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl of one to about four carbon atoms, straight chain or branched chain alkoxy of one to about four carbon atoms, and halogen, with the proviso that when the benzene ring is substituted by two such moieties, then the moieties together- contain no more than 6 carbon atoms; and each R₅ is independently selected from the group consisting of straight chain or branched chain alkoxy of one to about four-carbon atoms, halogen, and 30 straight chain or branched chain alkyl of one to about four carbon atoms, and n is an integer from zero to 2, with the proviso that if n is 2, then said R₃ groups together contain no more than 6 carbon atoms; 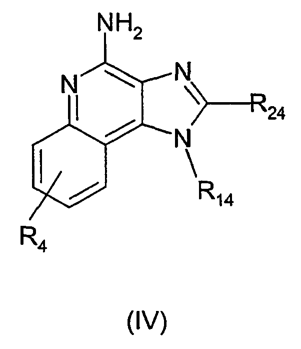 wherein
R₁₄ is -CHR_{A}R_{B} wherein R_{B} is hydrogen or a carbon-carbon bond, with the proviso that when R_{B} is hydrogen R_{A} is alkoxy of one to about four carbon atoms, hydroxyalkoxy of one to about four carbon atoms, 1-alkynyl of two to about ten carbon atoms, tetrahydropyranyl, alkoxyalkyl wherein the alkoxy moiety contains one to about four carbon atoms and the alkyl moiety contains one to about four carbon atoms, 2-, 3-, or 4-pyridyl, and with the further proviso that when R_{B} is a carbon-carbon bond R_{B} and R_{A} together form a tetrahydrofuranyl group optionally substituted with one or more substituents independently selected from the group consisting of hydroxy and hydroxyalkyl of one to about four carbon atoms; R₂₄ is selected from the group consisting of hydrogen, alkyl of one to about four carbon atoms, phenyl, and substituted phenyl wherein the substituent is selected from the group consisting of alkyl of one to about four carbon atoms, alkoxy of one to about four carbon atoms, and halogen; and R₄ is selected from the group consisting of hydrogen, straight chain or branched chain alkoxy containing one to about four carbon atoms, halogen, and straight chain or branched chain alkyl containing one to about four carbon atoms; 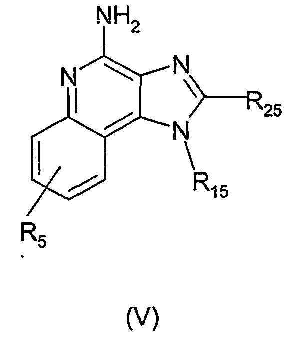 wherein
R₁₅ is selected from the group consisting of: hydrogen; straight chain or branched chain alkyl containing one to about ten carbon atoms and substituted straight chain or branched chain alkyl containing one to about ten carbon atoms, wherein the substituent is selected from the group consisting of cycloalkyl containing three to about six carbon atoms and cycloalkyl containing three to about six carbon atoms substituted by straight chain or branched chain alkyl containing one to about four carbon atoms; straight chain or branched chain alkenyl containing two to about ten carbon atoms and substituted straight chain or branched chain alkenyl containing two to about ten carbon atoms, wherein the substituent is selected from the group consisting of cycloalkyl containing three to about six carbon atoms and cycloalkyl containing three to about six carbon atoms substituted by straight chain or branched chain alkyl containing one to about four carbon atoms; hydroxyalkyl of one to about six carbon atoms; alkoxyalkyl wherein the alkoxy moiety contains one to about four carbon atoms and the alkyl moiety contains one to about six carbon atoms; acyloxyalkyl wherein the acyloxy moiety is alkanoyloxy of two to about four carbon atoms or benzoyloxy, and the alkyl moiety contains one to about six carbon atoms; benzyl; (phenyl)ethyl; and phenyl; said benzyl, (phenyl)ethyl or phenyl substituent being optionally substituted on the benzene ring by one or two moieties independently selected from the group consisting of alkyl of one to about four carbon atoms, alkoxy of one to about four carbon atoms, and halogen, with the proviso that when said benzene ring is substituted by two of said moieties, then the moieties together contain no more than six carbon atoms;
R₂₅ is 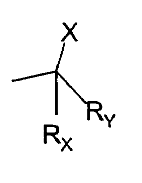 wherein
R_{X} and R_{Y} are independently selected from the group consisting of hydrogen, alkyl of one to about four carbon atoms, phenyl, and substituted phenyl wherein the substituent is elected from the group consisting of alkyl of one to about four carbon atoms, alkoxy of one to about four carbon atoms, and halogen; X is selected from the group consisting of alkoxy containing one to about four carbon atoms, alkoxyalkyl wherein the alkoxy moiety contains one to about four carbon atoms and the alkyl moiety contains one to about four carbon atoms, haloalkyl of one to about four carbon atoms, alkylamido wherein the alkyl group contains one to about four carbon atoms, amino, substituted amino wherein the substituent is alkyl or hydroxyalkyl of one to about four carbon atoms, azido, alkylthio of one to about four carbon atoms; and R₅ is selected from the group consisting of hydrogen, straight chain or branched chain alkoxy containing one to about four carbon atoms, halogen, and straight chain or branched chain alkyl containing one to about four carbon atoms; or a pharmaceutically acceptable salt of any of the foregoing.

Preferred alkyl groups are C₁ - C₄ alkyl, for example methyl, ethyl, propyl, 2-methylpropyl and butyl. Most preferred alkyl groups are methyl, ethyl and 2methyl-propyl. Preferred alkoxy groups are methoxy, ethoxy and ethoxymethyl.

The compounds recited above and methods for their preparation are disclosed in PCT patent application publication number WO 94/17043.

In instances where n can be zero, one, or two, n is preferably zero or one.

The substituents R₁-R₅ above are generally designated "benzo substituents" herein. The preferred benzo substituent is hydrogen.

The substituents R₁₁-R₁₅ above are generally designated "1-substituents" herein. The preferred 1-substituent is 2-methylpropyl or 2-hydroxy-2-methylpropyl.

The substituents R₂₁,-R₂₅ above are generally designated "2-substituents", herein. The preferred 2-substituents are hydrogen, alkyl of one to about six carbon atoms, alkoxyalkyl wherein the alkoxy moiety contains one to about four carbon atoms and the alkyl moiety contains one to about four carbon atoms. Most preferably the 2-substituent is hydrogen, methyl, or ethoxymethyl.

Particularly preferred is when the 1H-imidazo[4,5-c]quinolin-4-amine is a compound defined by formula VI below:

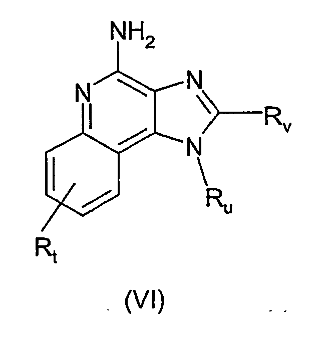

Wherein
Rₜ is selected from the group consisting of hydrogen, straight chain or branched chain alkoxy containing one to about four carbon atoms, halogen, and straight chain or branched chain alkyl containing one to about four carbon atoms;
Rᵤ is 2-methylpropyl or 2-hydroxy-2-methylpropyl; and
Rᵥ is hydrogen, alkyl of one to about six carbon atoms, or alkoxyalkyl wherein the alkoxy moiety contains one to about four carbon atoms and the alkyl moiety contains one to about four carbon atoms; or physiologically acceptable salts of any of the foregoing, where appropriate.

In formula VI, Rₜ is preferably hydrogen, Rᵤ is preferably 2-methylpropyl or 2-hydroxy-2-methylpropyl, and Rv is preferably hydrogen, methyl or ethoxymethyl.

Preferred 1H-imidazo[4,5-c]quinolin-4-amines include the following:
1-(2-methylpropyl)-1H-imidazo[4,5-c]quinolin-4-amine (a compound of formula VI wherein Rₜ is hydrogen, Rᵤ is 2-methylpropyl and Rᵥ is hydrogen);
1-(2-hydroxy-2-methylpropyl)-2-methyl-1H-imidazo[4,5-c]quinolin-4-amine (a compound of formula VI wherein Rₜ is hydrogen, Rᵤ is 2-hydroxy-2-methylpropyl, and Rᵥ is methyl;
1-(2-hydroxy-2-methylpropyl)-1H-imidazo[4,5-c]quinolin-4-amine (a compound of formula VI wherein Rₜ is hydrogen, Rᵤ is 2-hydroxy-2-methylpropyl, and Rᵥ is hydrogen)
1-(2-hydroxy-2-methylpropyl)-2-ethoxymethyl-1-H-imidazo[4,5-c]quinolin-4-amine (a compound of formula VI wherein Rₜ is hydrogen, Rᵤ is 2-hydroxy-2-methylpropyl and Rᵥ is ethoxymethyl);
or physiologically acceptable salts thereof.

It is possible for the vaccination methods and compositions according to the present application to be adapted for protection or treatment of mammals against a variety of disease states such as, for example, viral, bacterial or parasitic infections, cancer, allergies and autoimmune disorders. Some specific examples of disorders or disease states which can be protected against or treated by using the methods or compositions according to the present invention, are as follows:
Viral Infections
   Hepatitis viruses A, B, C, D & E, HIV, herpes viruses 1,2, 6 & 7, - cytomegalovirus, varicella zoster, papilloma virus, Epstein Barr virus, influenza viruses, para-influenza viruses, adenoviruses, coxsakie viruses, picorna viruses, rotaviruses, respiratory syncytial viruses, pox viruses, rhinoviruses, rubella virus, papovirus, mumps virus, measles virus.
Bacterial Infections
   Mycobacteria causing TB and leprosy,
   pneumocci, aerobic gram negative bacilli, mycoplasma, staphyloccocal infections, streptococcal infections, salmonellae, chlamydiae.
   Parasitic
   Malaria, leishmaniasis, trypanosomiasis, toxoplasmosis, schistosomiasis, filariasis,
Cancer
   Breast cancer, colon cancer, rectal cancer, cancer of the head and neck, renal cancer, malignant melanoma, laryngeal cancer, ovarian cancer, cervical cancer, prostate cancer.
Allergies
   Rhinitis due to house dust mite, pollen and other environmental allergens
Autoimmune disease
   Systemic lupus erythematosis

Preferably, the methods or compositions of the present invention are used to protect against or treat the viral disorders Hepatitis B, Hepatitis C, Human papilloma virus, Human immunodeficiency virus, or Herpes simplex virus; the bacterial disease TB; cancers of the breast, colon, ovary, cervix, and prostate; or the autoimmune diseases of asthma, rheumatoid arthritis and Alzheimer's

It is to be recognised that these specific disease states have been referred to by way of example only, and are not intended to be limiting upon the scope of the present invention.

The nucleotide sequences referred to in this application, which are to be expressed within a mammalian system, in order to induce an antigenic response, may encode for an entire protein, or merely a shorter peptide sequence which is capable of initiating an antigenic response. Throughout this specification and the appended claims, the phrase "antigenic peptide" or "immunogen" is intended to encompass all peptide or protein sequences which are capable of inducing an immune response within the animal concerned. Most preferably, however, the nucleotide sequence will encode for a full protein which is associated with the disease state, as the expression of full proteins within the animal system are more likely to mimic natural antigen presentation, and thereby evoke a full immune response. Some non-limiting examples of known antigenic peptides in relation to specific disease states include the following: Antigens which are capable of eliciting an immune response against a human pathogen, which antigen or antigenic composition is derived from HIV-1, (such as tat, nef, gp120 or gp160, gp40, p24, gag, env, vif, vpr, vpu, rev), human herpes viruses, such as gH, gL gM gB gC gK gE or gD or derivatives thereof or Immediate Early protein such as ICP27, ICP 47, IC P 4, ICP36 from HSV1 or HSV2, cytomegalovirus, especially Human, (such as gB or derivatives thereof), Epstein Barr virus (such as gp350 or derivatives thereof), Varicella Zoster Virus (such as gpI, II, III and IE63), or from a hepatitis virus such as hepatitis B virus (for example Hepatitis B Surface antigen or Hepatitis core antigen or pol), hepatitis C virus antigen and hepatitis E virus antigen, or from other viral pathogens, such as paramyxoviruses: Respiratory Syncytial virus (such as F and G proteins or derivatives thereof), or antigens from parainfluenza virus, measles virus, mumps virus, human papilloma viruses (for example HPV6, 11, 16, 18, eg L1, L2, E1, E2, E3, E4, E5, E6, E7), flaviviruses (e.g. Yellow Fever Virus, Dengue Virus, Tick-borne encephalitis virus, Japanese Encephalitis Virus) or Influenza virus cells, such as HA, NP, NA, or M proteins, or combinations thereof), or antigens derived from bacterial pathogens such as *Neisseria spp,* including *N. gonorrhea* and *N. meningitidis,* eg, transferrin-binding proteins, lactoferrin binding proteins, PilC, adhesins); *S. pyogenes* (for example M proteins or fragments thereof, C5A protease, *S. agalactiae, S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis* (for example ESAT6, Antigen 85A, -B or -C, MPT 44, MPT59, MPT45, HSP10,HSP65, HSP70, HSP 75, HSP90, PPD 19kDa [Rv3763], PPD 38kDa [Rv0934] ), *M. bovis, M. leprae, M. avium, M. paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E*. *coli,* enteropathogenic *E. coli* (for example shiga toxin-like toxin or derivatives thereof); *Vibrio spp,* including *V. cholera* (for example cholera toxin or derivatives thereof); *Shigella spp,* including S. *sonnei, S. dysenteriae, S. flexnerii; Yersinia* spp, including *Y.* enterocolitica (for example a Yop protein) , *Y. pestis, Y. pseudotuberculosis; Campylobacter spp,* including *C. jejuni* (for example toxins, adhesins and invasins) and *C. coli; Salmonella spp,* including *S. typhi, S. paratyphi, S. choleraesuis, S. enteritidis; Listeria spp.,* including *L. monocytogenes; Helicobacter spp,* including H. pylori (for example urease, catalase, vacuolating toxin); *Pseudomonas spp,* including *P. aeruginosa; Staphylococcus spp.,* including S. *aureus, S. epidermidis; Enterococcus spp.,* including E. *faecalis, E. faecium; Clostridium spp.,* including C. tetani (for example tetanus toxin and derivative thereof), *C. botulinum* (for example botulinum toxin and derivative thereof), *C. difficile* (for example clostridium toxins A or B and derivatives thereof); *Bacillus spp.,* including *B. anthracis* (for example botulinum toxin and derivatives thereof); *Corynebacterium spp.,* including C. *diphtheriae* (for example diphtheria toxin and derivatives thereof); *Borrelia spp.,* including B. *burgdorferi* (for example OspA, OspC, DbpA, DbpB), B. *garinii* (for example OspA, OspC, DbpA, DbpB), *B. afzelii* (for example OspA, OspC, DbpA, DbpB), B. *andersonii* (for example OspA, OspC, DbpA, DbpB), B. *hermsii*; *Ehrlichia spp.,* including E. *equi.* and the agent of the Human Granulocytic Ehrlichiosis; *Rickettsia spp,* including R. *rickettsii; Chlamydia spp., including C*. *trachomatis* (for example MOMP, heparin-binding proteins), *C*. *pneumoniae* (for example MOMP, heparin-binding proteins), *C*. *psittaci; Leptospira spp.,* including *L. interrogans; Treponema spp.,* including *T. pallidum* (for example the rare outer membrane proteins), T. *denticola*, *T. hyodysenteriae*; or derived from parasites such as *Plasmodium spp.,* including P. *falciparum; Toxoplasma spp.,* including *T. gondii (for example* SAG2, SAG3, *Tg34); Entamoeba spp.,* including E. *histolytica; Babesia spp.,* including B. *microti; Trypanosoma spp.,* including T. *cruzi; Giardia spp.,* including G. *lamblia; Leshmania spp.,* including L. *major; Pneumocystis spp.,* including P. *carinii; Trichomonas spp.,* including T. *vaginalis; Schisostoma spp.,* including S. *mansoni,* or derived from yeast such as *Candida* spp., including C. *albicans; Cryptococcus spp.,* including C. *neoformans.*

Other preferred specific antigens for *M*. *tuberculosis* are for example Rv2557, Rv2558, RPFs: Rv0837c, Rv1884c, Rv2389c, Rv2450, Rv1009, aceA (Rv0467), PstS1, (Rv0932), SodA (Rv3846), Rv2031c 16kDal., Tb Ra12, Tb H9, Tb Ra35, Tb38-1, Erd 14, DPV, MTI, MSL, mTTC2 and hTCC1 (WO 99/51748). Proteins for *M*. *tuberculosis* also include fusion proteins and variants thereof where at least two, preferably three polypeptides of *M*. *tuberculosis* are fused into a larger protein. Preferred fusions include Ra12-TbH9-Ra35, Erd14-DPV-MTI, DPV-MTI-MSL, Erd14-DPV-MTI-MSL-mTCC2, Erd14-DPV-MTI-MSL, DPV-MTI-MSL-mTCC2, TbH9-DPV-MTI (WO 99/51748).

Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial vaccines comprise antigens derived from *Streptococcus spp,* including *S*. *pneumoniae* (PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy variants or fusion proteins thereof.

The antigens that may be used in the present invention may further comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. Its full structure is disclosed in the International Patent Application No.

PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a Malaria vaccine wherein the antigenic preparation comprises a combination of the RTS, S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.

The invention contemplates the use of an anti-tumour antigen and be useful for the immunotherapeutic treatment of cancers. For example, tumour rejection antigens such as those for prostrate, breast, colorectal, lung, pancreatic, renal or melanoma cancers. Exemplary antigens include MAGE 1 , 3 and MAGE 4 or other MAGE antigens such as disclosed in WO99/40188, PRAME, BAGE, Lage (also known as NY Eos 1) SAGE and HAGE (WO 99/53061) or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma.

MAGE antigens for use in the present invention may be expressed as a fusion protein with an expression enhancer or an Immunological fusion partner. In particular, the Mage protein may be fused to Protein D from Heamophilus influenzae B. In particular, the fusion partner may comprise the first 1/3 of Protein D. Such constructs are disclosed in Wo99/40188. Other examples of fusion proteins that may contain cancer specific epitopes include *bcr* / *abl* fusion proteins.

In a preferred embodiment prostate antigens are utilised, such as Prostate specific antigen (PSA), PAP, PSCA (PNAS 95(4) 1735 -1740 1998), PSMA or antigen known as Prostase.

Prostase is a prostate-specific serine protease (trypsin-like), 254 amino acid-long, with a conserved serine protease catalytic triad H-D-S and a amino-terminal pre-propeptide sequence, indicating a potential secretory function (P. Nelson, Lu Gan, C. Ferguson, P. Moss, R. Gelinas, L. Hood & K. Wand, "Molecular cloning and characterisation of prostase, an androgen-regulated serine protease with prostate restricted expression, In Proc. Natl. Acad. Sci. USA (1999) 96, 3114-3119). A putative glycosylation site has been described. The predicted structure is very similar to other known serine proteases, showing that the mature polypeptide folds into a single domain. The mature protein is 224 amino acids-long, with one A2 epitope shown to be naturally processed.

Prostase nucleotide sequence and deduced polypeptide sequence and homologs are disclosed in Ferguson, et al. (Proc. Natl. Acad. Sci. USA 1999, 96, 3114-3119) and in International Patent Applications No. WO 98/12302 (and also the corresponding granted patent US 5,955,306), WO 98/20117 (and also the corresponding granted patents US 5,840,871 and US 5,786,148) (prostate-specific kallikrein) and WO 00/04149 (P703P).
The present invention provides antigens comprising prostase protein fusions based on prostase protein and fragments and homologues thereof ("derivatives"). Such derivatives are suitable for use in therapeutic vaccine formulations which are suitable for the treatment of a prostate tumours. Typically the fragment will contain at least 20, preferably 50, more preferably 100 contiguous amino acids as disclosed in the above referenced patent and patent applications.

A further preferred prostate antigen is known as P501S, sequence ID no 113 of WO98/37814. Immunogenic fragments and portions encoded by the gene thereof comprising at least 20, preferably 50, more preferably 100 contiguous amino acids as disclosed in the above referenced patent application, are contemplated. A particular fragment is PS108 (WO 98/50567).

Other prostate specific antigens are known from Wo98/37418, and WO/004149. Another is STEAP PNAS 96 14523 14528 7 -12 1999.

Other tumour associated antigens useful in the context of the present invention include: Plu -1 J Biol. Chem 274 (22) 15633 -15645, 1999, HASH -1, HasH-2, Cripto (Salomon et al Bioessays 199, 21 61 -70,US patent 5654140) Criptin US patent 5 981 215, ., Additionally, antigens particularly relevant for vaccines in the therapy of cancer also comprise tyrosinase and survivin.

The present invention is also useful in combination with breast cancer antigens such as Muc-1, Muc-2, EpCAM, her 2/ Neu, mammaglobin (US patent 5668267) or those disclosed in WO/00 52165, WO99/33869, WO99/19479, WO 98/45328. Her 2 neu antigens are disclosed inter alia, in US patent 5,801,005. Preferably the Her 2 neu comprises the entire extracellular domain ( comprising approximately amino acid 1 -645) or fragments thereof and at least an immunogenic portion of or the entire intracellular domain approximately the C terminal 580 amino acids . In particular, the intracellular portion should comprise the phosphorylation domain or fragments thereof. Such constructs are disclosed in WO00/44899. A particularly preferred construct is known as ECD PD a second is known as ECD □PD. (See WO/00/44899.)

The her 2 neu as used herein can be derived from rat, mouse or human.

The vaccine may also contain antigens associated with tumour-support mechanisms (e.g. angiogenesis, tumour invasion) for example tie 2, VEGF.

Vaccines of the present invention may also be used for the prophylaxis or therapy of chronic disorders in addition to allergy, cancer or infectious diseases. Such chronic disorders are diseases such as asthma, atherosclerosis, and Alzheimers and other auto-immune disorders. Vaccines for use as a contraceptive may also be considered.

Antigens relevant for the prophylaxis and the therapy of patients susceptible to or suffering from Alzheimer neurodegenerative disease are, in particular, the N terminal 39 -43 amino acid fragment (ABthe amyloid precursor protein and smaller fragments. This antigen is disclosed in the International Patent Application No. WO 99/27944 - (Athena Neurosciences).

Potential self-antigens that could be included as vaccines for auto-immune disorders or as a contraceptive vaccine include: cytokines, hormones, growth factors or extracellular proteins, more preferably a 4-helical cytokine, most preferably IL13. Cytokines include, for example, IL1, IL2, IL3, IL4, IL5, IL6, IL7, IL8, IL9, IL10, IL11, IL12, IL13, IL14, IL15, IL16, IL17, IL18, IL20, IL21, TNF, TGF, GMCSF, MCSF and OSM. 4-helical cytokines include IL2, IL3, IL4, IL5, IL13, GMCSF and MCSF. Hormones include, for example, luteinising hormone (LH), follicle stimulating hormone (FSH), chorionic gonadotropin (CG), VGF, GHrelin, agouti, agouti related protein and neuropeptide Y. Growth factors include, for example, VEGF.
The vaccines of the present invention are particularly suited for the immunotherapeutic treatment of diseases, such as chronic conditions and cancers, but also for the therapy of persistent infections. Accordingly the vaccines of the present invention are particularly suitable for the immunotherapy of infectious diseases, such as Tuberculosis (TB), HIV infections such as AIDS and Hepatitis B (HepB) virus infections.

In a particularly preferred embodiment the nucleic acid encodes one or more of the following antigens:-
HBV - PreS1 PreS2 and Surface env proteins, core and pol
HIV - gp120 gp40, gp160, p24, gag, pol, env, vif, vpr, vpu, tat, rev, nef
Papilloma - E1, E2, E3, E4, E5, E6, E7, E8, L1, L2
HSV - gL, gH, gM, gB, gC, gK, gE, gD, ICP47, ICP36, ICP4
Influenza - haemaggluttin, nucleoprotein
TB - Mycobacterial super oxide dismutase, 85A, 85B, MPT44, MPT59, MPT45, HSP10, HSP65, HSP70, HSP90, PPD 19kDa Ag, PPD 38kDa Ag.

The nucleotide sequence may be RNA or DNA including genomic DNA, synthetic DNA or cDNA. Preferably the nucleotide sequence is a DNA sequence and most preferably, a cDNA sequence. In order to obtain expression of the antigenic peptide within mammalian cells, it is necessary for the nucleotide sequence encoding the antigenic peptide to be presented in an appropriate vector system. By "appropriate vector" as used herein is meant any vector that will enable the antigenic peptide to be expressed within a mammal in sufficient quantities to evoke an immune response.

For example, the vector selected may comprise a plasmid, promoter and polyadenylation/ transcriptional termination sequence arranged in the correct order to obtain expression of the antigenic peptides. The construction of vectors which include these components and optionally other components such as enhancers, restriction enzyme sites and selection genes, such as antibiotic resistance genes, is well known to persons skilled in the art and is explained in detail in Maniatis et al "Molecular Cloning: A Laboratory Manual", Cold Spring Harbour Laboratory, Cold Spring Harbour Press, Vols 1-3, 2nd Edition, 1989.

As it is preferred to prevent the plasmids replicating within the mammalian host and integrating within the chromosomal DNA of the animal, the plasmid will preferably be produced without an origin of replication that is functional in eukaryotic cells.

The methods and compositions according to the present invention can be used in relation to prophylactic or treatment procedures of all mammals including, for example, domestic animals, laboratory animals, farm animals, captive wild animals and, most preferably, humans.

The present inventors have demonstrated that 1H-imidazo[4,5-c]quinolin-4-amine derivatives when used as adjuvants in DNA vaccination are capable of enhancing both Th1 and Th2 cytokine profiles. The term adjuvant or adjuvant component as used herein is intended to convey that the derivatives or component comprising the derivatives act to enhance and/or alter the body's response to an immunogen in a desired fashion. So, for example, an adjuvant may be used to shift an immune response to a predominately Th1 response, or to increase both types of responses.

A preferential inducer of a TH1 type of immune response enables a cell mediated response to be generated. High levels of Th1-type cytokines tend to favour the induction of cell mediated immune responses to the given antigen, whilst high levels of Th2-type cytokines tend to favour the induction of humoral immune responses to the antigen.

It is important to remember that the distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of II-4, IL-5, IL-6, IL-10.

The immunogen component comprising a vector which comprises the nucleotide sequence encoding an antigenic peptide can be administered in a variety of manners. It is possible for the vector to be administered in a naked form (that is as naked nucleotide sequence not in association with liposomal formulations, with viral vectors or transfection facilitating proteins) suspended in an appropriate medium, for example a buffered saline solution such as PBS and then injected intramuscularly, subcutaneously, intraperitonally or intravenously, although some earlier data suggests that intramuscular or subcutaneous injection is preferable (Brohm et al Vaccine 16 No. 9/10 pp 949-954 (1998), the disclosure of which is included herein in its entirety by way of reference). It is additionally possible for the vectors to be encapsulated by, for example, liposomes or within polylactide co-glycolide (PLG) particles (25) for administration via the oral, nasal or pulmonary routes in addition to the routes detailed above.

It is also possible, according to a preferred embodiment of the invention, for intradermal administration of the immunogen component, preferably via use of gene-gun (particularly particle bombardment) administration techniques. Such techniques may involve coating of the immunogen component on to gold beads which are then administered under high pressure into the epidermis, such as, for example, as described in Haynes et al J. Biotechnology 44: 37-42 (1996).

The vectors which comprise the nucleotide sequences encoding antigenic peptides are administered in such amount as will be prophylactically or therapeutically effective. The quantity to be administered, is generally in the range of one picogram to 1 milligram, preferably 1 picogram to 10 micrograms for particle-mediated delivery, and 10 micrograms to 1 milligram for other routes of nucleotide per dose. The exact quantity may vary considerably depending on the species and weight of the mammal being immunised, the route of administration, the potency and dose of the 1H-imidazo-[4,5-c]quinolin derivative, the nature of the disease state being treated or protected against, the capacity of the subject's immune system to produce an immune response and the degree of protection or therapeutic efficacy desired. Based upon these variables, a medical or veterinary practitioner will readily be able to determine the appropriate dosage level.

It is possible for the immunogen component comprising the nucleotide sequence encoding the antigenic peptide, to be administered on a once off basis or to be administered repeatedly, for example, between 1 and 7 times, preferably between 1 and 4 times, at intervals between about 1 day and about 18 months. Once again, however, this treatment regime will be significantly varied depending upon the size and species of animal concerned, the disease which is being treated/protected against, the amount of nucleotide sequence administered, the route of administration, the potency and dose of 1 H-imidazo[4,5-c]quinolin-4-amine derivatives selected and other factors which would be apparent to a skilled veterinary or medical practitioner.

The adjuvant component specified herein can similarly be administered via a variety of different administration routes, such as for example, via the oral, nasal, pulmonary, intramuscular, subcutaneous, intradermal or topical routes. Preferably, the component is administered via the intradermal or topical routes. This administration may take place between about 14 days prior to and about 14 days post administration of the nucleotide sequence, preferably between about 1 day prior to and about 3 days post administration of the nucleotide sequence. Most preferred is when the adjuvant component is administered substantially simultaneously with the administration of the nucleotide sequence. By "substantially simultaneous" what is meant is that administration of the adjuvant component is preferably at the same time as administration of the nucleotide sequence, or if not, at least within a few hours either side of nucleotide sequence administration. In the most preferred treatment protocol, the adjuvant component will be administered substantially simultaneously to administration of the nucleotide sequence. Obviously, this protocol can be varied as necessary, in accordance with the type of variables referred to above.

Once again, depending upon such variables, the dose of administration of the derivative will also vary, but may, for example, range between about 0.1 mg per kg to about 100mg per kg, where "per kg" refers to the body weight of the mammal concerned. This administration of the 1H-imidazo[4,5-c]quinolin-4-amine derivative would preferably be repeated with each subsequent or booster administration of the nucloetide sequence. Most preferably, the administration dose will be between about 1 mg per kg to about 50mg per kg.

While it is possible for the adjuvant component to comprise only 1H-imidazo[4,5-c]quinolin-4-amine derivatives to be administered in the raw chemical state, it is preferable for administration to be in the form of a pharmaceutical formulation. That is, the adjuvant component will preferably comprise the 1H-imidazo[4,5-c]quinolin-4-amine combined with one or more pharmaceutically or veterinarily acceptable carriers, and optionally other therapeutic ingredients. The carrier(s) must be "acceptable" in the sense of being compatible with other ingredients within the formulation, and not deleterious to the recipient thereof. The nature of the formulations will naturally vary according to the intended administration route, and may be prepared by methods well known in the pharmaceutical art. All methods include the step of bringing into association a 1H-imidazo[4,5-c]quinolin-4-amine derivative with an appropriate carrier or carriers. In general, the formulations are prepared by uniformly and intimately bringing into association the derivative with liquid carriers or finely divided solid carriers, or both, and then, if necessary, shaping the product into the desired formulation. Formulations of the present invention suitable for oral administration may be presented as discrete units such as capsules, cachets or tablets each containing a pre-determined amount of the active ingredient; as a powder or granules; as a solution or a suspension in an aqueous liquid or a non-aqueous liquid; or as an oil-in-water liquid emulsion or a water-in-oil emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent.

The tablets may optionally be coated or scored and may be formulated so as to provide slow or controlled release of the active ingredient.

Formulations for injection via, for example, the intramuscular, intraperitoneal, or subcutaneous administration routes include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze-dried (lyophilised) condition requiring only the addition of the sterile liquid carrier, for example, water for injections, immediately prior to use. Extemporaneous injection solutions and suspensions may be prepared from sterile powders, granules and tablets of the kind previously described. Formulations suitable for pulmonary administration via the buccal or nasal cavity are presented such that particles containing the active ingredient, desirably having a diameter in the range of 0.5 to 7 microns, are delivered into the bronchial tree of the recipient. Possibilities for such formulations are that they are in the form of finely comminuted powders which may conveniently be presented either in a piercable capsule, suitably of, for example, gelatine, for use in an inhalation device, or alternatively, as a self-propelling formulation comprising active ingredient, a suitable liquid propellant and optionally, other ingredients such as surfactant and/or a solid diluent. Self-propelling formulations may also be employed wherein the active ingredient is dispensed in the form of droplets of a solution or suspension. Such self-propelling formulations are analogous to those known in the art and may be prepared by established procedures. They are suitably provided with either a manually-operable or automatically functioning valve having the desired spray characteristics; advantageously the valve is of a metered type delivering a fixed volume, for example, 50 to 100 µL, upon each operation thereof.

In a further possibility, the adjuvant component may be in the form of a solution for use in an atomiser or nebuliser whereby an accelerated airstream or ultrasonic agitation is employed to produce a find droplet mist for inhalation.

Formulations suitable for intranasal administration generally include presentations similar to those described above for pulmonary administration, although it is preferred for such formulations to have a particle diameter in the range of about 10 to about 200 microns, to enable retention within the nasal cavity. This may be achieved by, as appropriate, use of a powder of a suitable particle size, or choice of an appropriate valve. Other suitable formulations include coarse powders having a particle diameter in the range of about 20 to about 500 microns, for administration by rapid inhalation through the nasal passage from a container held close up to the nose, and nasal drops comprising about 0.2 to 5% w/w of the active ingredient in aqueous or oily solutions. In one embodiment of the invention, it is possible for the vector which comprises the nucleotide sequence encoding the antigenic peptide to be administered within the same formulation as the 1H-imidazo[4,5-c]quinolin-4-amine derivative.

Hence in this embodiment, the immunogenic and the adjuvant component are found within the same formulation.

In a preferred embodiment the adjuvant component is prepared in a form suitable for gene-gun administration, and is administered via that route substantially simultaneous to administration of the nucleotide sequence. For preparation of formulations suitable for use in this manner, it may be necessary for the 1H-imidazo[4,5-c]quinolin-4-amine derivative to be lyophilised and adhered onto, for example, gold beads which are suited for gene-gun administration.

In an alternative embodiment, the adjuvant component may be administered as a dry powder, via high pressure gas propulsion. This will preferably be substantially simultaneous to administration of the nucleotide sequence.

Even if not formulated together, it may be appropriate for the adjuvant component to be administered at or about the same administration site as the nucleotide sequence.

Other details of pharmaceutical preparations can be found in Remington's Pharmaceutical Sciences, Mack Publishing Company, Easton, Pennysylvania (1985), the disclosure of which is included herein in its entirety, by way of reference.

The present invention will now be described further, with reference to the following non-limiting examples:

### Examples

### 1. Imiquimod increases the magnitude of the cytotoxic T cell response to a nucleic acid vaccine.

### Construction of plasmids and DNA preparation

The plasmids used are based upon pVAC1, obtained from Michelle Young, GlaxoWellcome, UK, a modification of the mammalian expression vector, pCl, (Promega), where the multiple cloning site, from EcoRI to Bst ZI, has been replaced by the EMCV IRES sequence flanked 5' by unique Nhe I, Rsr II and Xho I and 3' by unique Pac I, Asc I and Not I restriction enzyme sites.

An influenza nucleoprotein expression plasmid, pVAC1.PR, was constructed by ligating PCR amplified cDNA encoding nucleoprotein of influenza A virus strain PR/8/34 from pAR501, (a gift from Dr. D. Kiossis, NIMR, London, UK), into the expression vector pVAC1.

Plasmid DNA was propagated in E. coli, and prepared using plasmid purification kits (QIAGEN Ltd, Crawley, UK), and stored at -20°C at approximately 1 mg plasmid DNA/ml in 10 mM Tris/EDTA buffer.

### Preparations of cartridges for DNA immunisation

Preparation of cartridges for the Accell gene transfer device was as previously described (Eisenbraun et al DNA and Cell Biology, 1993 Vol 12 No 9 pp 791-797; Pertner et al). Briefly, plasmid DNA was coated onto 2 µm gold particles (DeGussa Corp., South Plainfield, N.J., USA) and loaded into Tefzel tubing, which was subsequently cut into 1.27 cm lengths to serve as cartridges and stored desiccated at 4°C until use. In a typical vaccination, each cartridge contained 0.5 mg gold coated with -0.05 µg pVAC1.PR with empty vector (pVAC1) added to provide a total of 0.5 µg DNA/cartridge.

### Immunisations

To examine whether imiquimod could increase the cytotoxic T cell response generated by nucleic acid vaccination, pVAC1.PR was administered by particle mediated gene transfer (0.05 µg/cartridge) into the skin of mice. Plasmid was delivered to the shaved target site of abdominal skin of C57BI/6 mice (purchased from Charles River United Kingdom Ltd, Margate, UK) from two cartridges using the Accell gene transfer device at 500 Ib/in2 (McCabe WO 95/19799). ) Immediately following vaccination imiquimod (prepared as a suspension in vehicle which comprised 0.3%(w/v) methylcellulose and 0.1% (v/v) Tween in sterile water) was administered by a single subcutaneous injection (0.05ml/10g body weight formulated to provide a dose of 30mg/kg)) at the immunisation site. Plasmid and imiquimod controls were empty vector (pVAC1) and vehicle, respectively.

### Cytotoxic T cell responses

The cytotoxic T cell response was assessed by CD8+ T cell-restricted IFN-γ ELISPOT assay of splenocytes collected one week later. Mice were killed by cervical dislocation and spleens were collected into ice-cold PBS. Splenocytes were teased out into phosphate buffered saline (PBS) followed by lysis of red blood cells (1 minute in buffer consisting of 155mM NH₄Cl, 10 mM KHCO₃, 0.1mM EDTA). After two washes in PBS to remove particulate matter the single cell suspension was aliquoted into ELISPOT plates previously coated with capture IFN-γ antibody and stimulated with CD8-restricted cognate peptide. After overnight culture, IFN-γ producing cells were visualised by application of anti-murine IFN-γ-biotin labelled antibody (Pharmingen) followed by streptavidin - conjugated alkaline phosphatase and quantitated using image analysis.

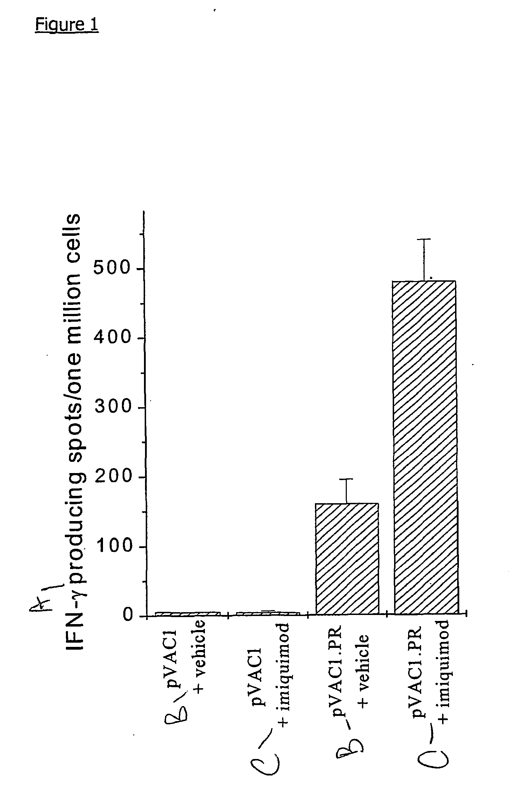

The results of this experiment (Figure 1) show that the number of cytotoxic T cells in the spleens of mice treated with the combination of pVAC1.PR and imiquimod was 3 times greater than with pVAC1.PR+ vehicle alone. No difference was seen between the control plasmid (pVAC1) + imiquimod or vehicle groups indicating that the effect of imiquimod was antigen-restricted. These results clearly show that imiquimod is a potent adjuvant for nucleic acid vaccination.

### 2. Imiquimod increases the magnitude of the CD4+ T cell response to a nucleic acid vaccine

### Plasmids, DNA and cartridge preparation

A chicken ovalbumin expression plasmid, pVAC1.OVA was constructed by ligating PCR amplified cDNA encoding chicken ovalbumin from pUGOVA (a gift from Dr. F. Carbone) into the expression vector pVAC1 and cartridges were prepared (as described in example 1 above).

### Mice and immunisations

Male or female D0.11.10 transgenic mice (6-10 weeks old) were bred in our specific pathogen-free animal breeding facilities at Bury Green Farm. The transgene that these mice express is the T cell receptor (TCR) specific for a chicken ovalbumin peptide residue (residues 323-339; OVA peptide) bound to MHC-II molecule (I-A^{d}). The monoclonal antibody, KJ1-26 which specifically recognises this TCR is used for identification of TCR-transgenic T cells. Examination of a number of these mice shows that a large proportion (40-65%) of the CD4⁺ T cells are KJ1-26⁺, although a very small population of CD4⁻ CD8⁺ KJ1.26⁺ T cells are also present (Pape, et al Immunological Reviews (1997) 156: 67-78).

Balb/c mice were purchased from Charles River United Kingdom Ltd. (Margate, UK).

CD4+ T cell responses were examined using an adoptive transfer model which enhances the sensitivity of the immune parameters to be measured. Here, T cells which specifically recognise a peptide sequence from ovalbumin protein were adoptively transferred from transgenic into naïve wild-type mice before immunisation. Briefly, 24 hours before immunisation, D0.11.10 splenocytes were adoptively transferred into Balb/c mice at 6-8 weeks of age. Splenocytes were prepared as in example 1 above. Cells were then adoptively transferred into the lateral tail vein by injection of 100µl (i.e. 25 x 10⁶ splenocytes/mouse).

Mice were subsequently immunised by particle mediated gene transfer with a plasmid encoding ovalbumin (pVAC1.OVA; 0.5 µg/cartridge) and imiquimod (30 mg/kg), or with control plasmid or vehicle, as in example 1.

### CD4 T cell responses

Mice were killed by cervical dislocation and inguinal and periaortic lymph nodes were collected and prepared as for splenocytes (described in example 1), except that the red blood cell lysis step was omitted.

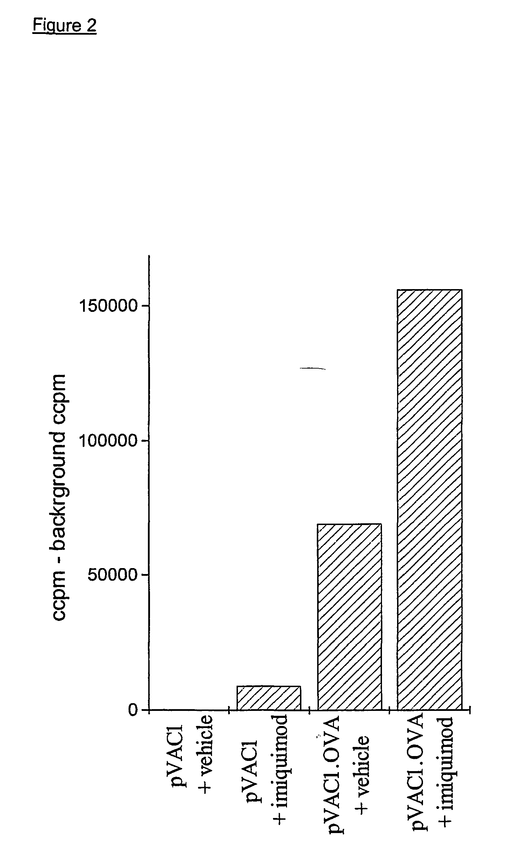

To measure the clonal expansion of CD4+ T cells following immunisation, proliferation of ovalbumin-specific T cells following re-challenge in vitro with cognate ovalbumin peptide, was assessed in lymph node preparations collected 3 days after immunisation. Proliferation, assessed by up take of tritiated thymidine by proliferating cells, was more than twice that with the combination of pVAC1.OVA + imiquimod compared with pVAC1.OVA + vehicle (Figure 2). No difference was seen between the control plasmid (pVAC1) + imiquimod or vehicle groups indicating that the effect of imiquimod was antigen-restricted.

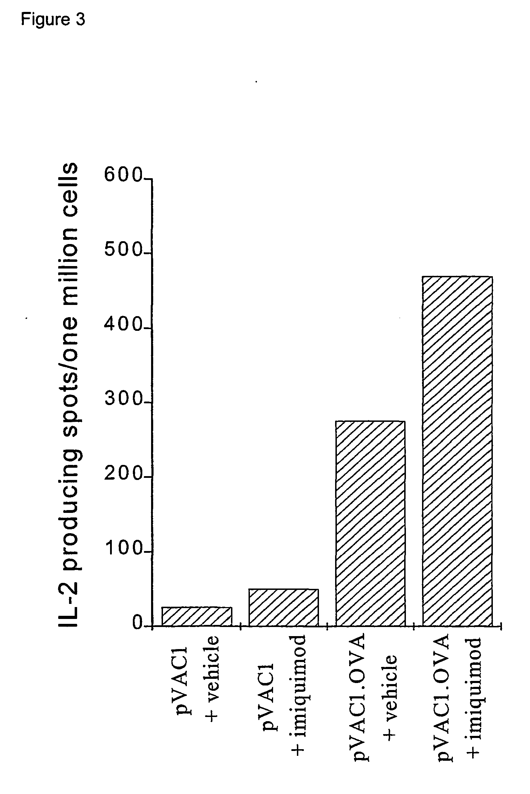

A substantial increase in activation of CD4 T cells, assessed by IL-2 ELISPOT (example 1, above) on lymph node cells collected 6 days after immunisation (Figure 3), was found for the combination of pVAC1.OVA + imiquimod compared with pVAC1.OvA alone (70% increase).

These results show that the adjuvant effect of imiquimod in vivo extends to both (ie. CD8+ cytotoxic and CD4+ helper) T cell populations. They also confirm the potency of the adjuvant effect of imiquimod for nucleic acid vaccination.

### 3. Imiquimod induces both Th1 and Th2 responses to a nucleic acid vaccine.

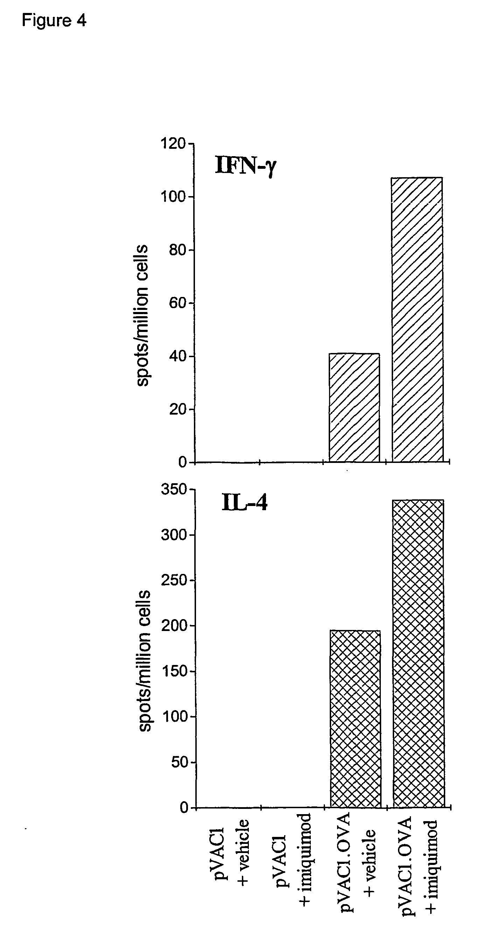

Th CD4+ subsets were assessed using the adoptive transfer model (example 2, above). IFN-γ-producing (Th1) and IL-4-producing (Th2) cells were assayed by ELISPOT (example 1, above). Imiquimod induced an increase in both cytokine-producing cells, although the increase was more substantial for IFN-γ compared with IL-4 (Figure 4). No cytokine producing cells were detected in empty vector immunised controls. The bias towards IFN-γ-producing cells, indicates that imiquimod not only acts as an adjuvant for nucleic acid vaccination but that it preferentially induces a Th1 type of response, the latter being substantiated by example 1 above.

### 4. Effect of Imiquimod on Humoral Immune Responses

To examine whether imiquimod increases the humoral response generated by nucleic acid vaccination, a plasmid encoding an antigen (eg. nucleoprotein from influenza virus) is administered by PMGT into the skin of mice. A primary immunisation is followed by one or more boost immunisations, with at least 4 weeks between each immunisation. Before and/or immediately following each immunisation imiquimod is administered by a single subcutaneous injection at the immunisation site. Plasmid and imiquimod controls are empty vector (ie. without antigen) and vehicle, respectively. Blood samples are collected from the tail vein 1 to 3 days prior to, and at intervals after, immunisation. Serum is separated and stored at -20°C for subsequent antibody analysis.

The humoral response is assessed by measuring antigen-specific whole IgG antibody levels (eg. nucleoprotein from influenza virus) in the serum samples collected after primary and boost immunisation. Microtitre plates (Nunc Immunoplate F96 maxisorp, Lifet Technologies) are coated with 10µg/ml antigen by overnight incubation at 4°C and washed 4 times with washing buffer (PBS containing 5% Tween 20 and 0.1%-sodium azide). This is followed by a 1 hour incubation at 20°C with serum samples serially diluted in blocking buffer. After 4 further washes (as above) to remove unbound antibody, plates are incubated for 1 hour with peroxidase conjugated anti-mouse IgG antibody (Southern Biotechnology) diluted in blocking buffer. The amount of bound antibody is determined after 4 further washes (as above) followed by addition of TMB substrate solution (T-8540-Sigma). After 30 minutes at 20°Cprotected from light, the reaction is stopped with 1M sulphuric acid and absorbance read at 450 nm. Titres are defined as the highest dilution to reach an OD of 0.2. An increase in antibody levels in immunised mice treated with imiqimod over those immunised without imiquimod indicates an enhancing effect of imiquimod on humoral responses.

### 5. Imiquimod enhances immune response to HIV antigens

### Plasmids, DNA and cartridge preparation

A plasmid expressing the Gag and Nef antigens (ie.WRG7077Gag/Nef) was constructed based on WRG7077. The original WRG7077 plasmid was constructed by replacing the beta-lactamase gene containing Eam1105I- Pstl fragment of pUC19 (available from Amersham Pharmacia Biotech UK Ltd., Amersham Place, Little Chalfont, Bucks, HP7 9NA) with an EcoRI fragment of pUC4K (Amersham-Pharmacia) containing the Kanamycin resistance gene, following blunt ending of both fragments using T4 DNA polymerase. The human Cytomegalovirus IE1 promoter/enhancer, intron A, was derived from plasmid JW4303 obtained from Dr Harriet Robinson, University of Massachussets, and inserted into the Sal1 site of pUC19 as a Xhol -Sal1 fragment, incorporating the bovine growth hormone polyadenylation signal. The Gag-Nef fusion was generated by PCR stitching of a truncated Nef with 195bp deleted from the 5' end of the gene removing the first 65 amino acids, derived from HIV-1 strain 248A (Genbank Acc. No. L15518, a kink gift from G. Thompson), and p17p24 (Gag) from the plasmid pHXBΔPr (Maschera *et al.,* 1995) containing HIV-1 clade B strain HXB2 (Genbank Acc. No. K03455). The resulting Gag-Nef fusion was subsequently Ligated into WRG7077 as a Notl-BamHI fragment. Plasmid DNA and cartridge preparation was as described in example 1.

### Mice and immunisations

Balb/c mice were immunised, and imiquimod prepared and administered, as described in example 1. Here mice received a primary immunisation followed by a boost immunisation 42 days later. Imiquimod (100 mg/kg) was administered at the boost only.

### Cytotoxic T cell responses

IFN-γ ELISPOT assays were performed as described (example 1), using CD8-restricted cognate peptides for the Gag and Nef antigens to stimulate splenocytes collected 5 days after the boost immunisation.

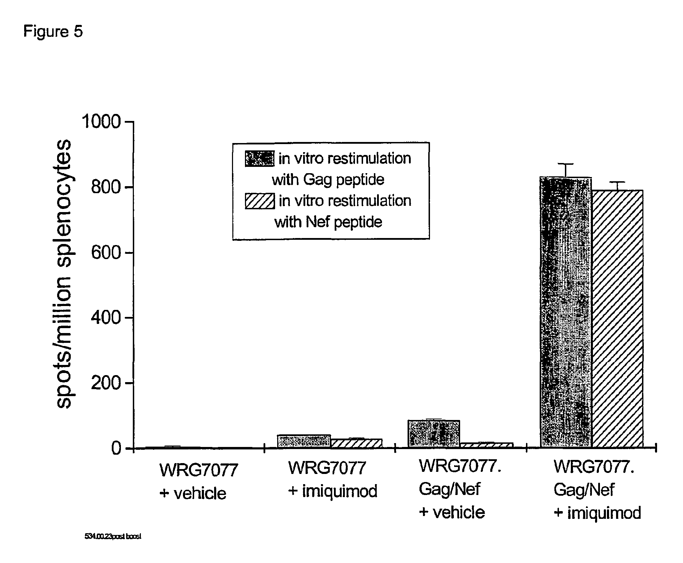

A substantial increase in cytotoxic T cells was observed when imiquimod was co-administered compared with the group that received plasmid with vehicle alone (10 fold and 100 fold for Gag and Nef, respectively) (Figure 5). These findings suggest that the adjuvant effect of imiquimod is not antigen specific and exemplify efficacy with viral antigens, most specifically for HIV.

### 6. Analogues of imiquimod are effective adjuvants for nucleic acid vaccination.

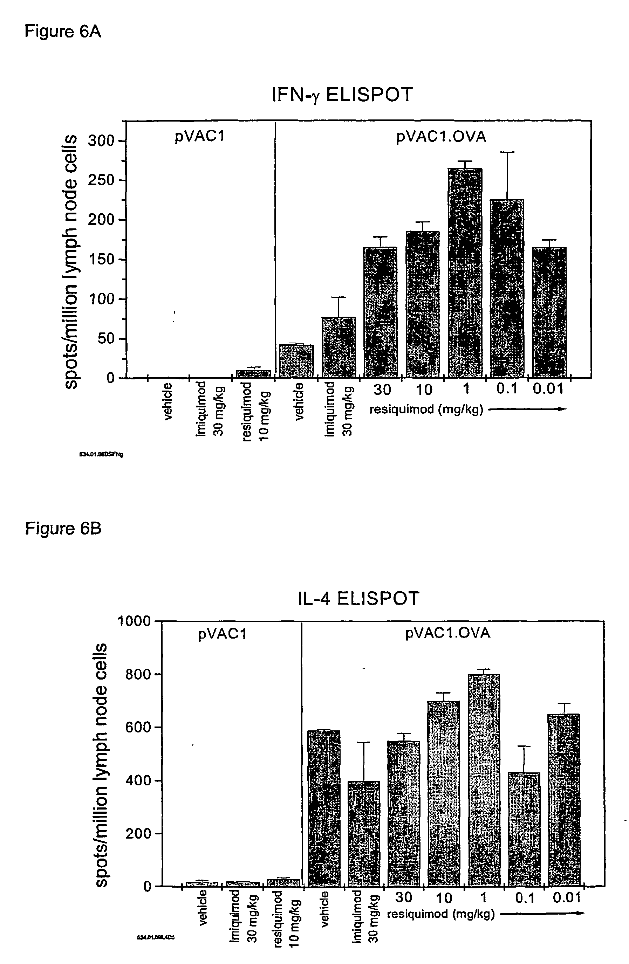

Cartridges were prepared using the pVAC1.OVA plasmid, immunisations and T cell responses were as described in example 2. Analogues of imiquimod tested were 1-(2-hydroxy-2-methylpropyl)-2-ethoxymethyl-1-H-imidazo[4,5-c]quinolin-4-amine (ie. resiquimod), 1-(2-hydroxy-2-methylpropyl)-2-methyl-1H-imidazo[4,5-c]quinolin-4-amine and 1-(2,hydroxy-2-methylpropyl)-1H-imidazo[4,5-c]quinolin-4-amine. Resiquimod was tested over a broad range of doses, with 1 mg/kg apparently optimal. Here analysis of lymph node cells collected 5 days after immunisations showed a six fold increase of IFN-γ producing CD4 T cells, with only a modest effect on IL-4 producing CD4 T cells (Figure 6), further supporting the Th1 biased effect observed in example 3.

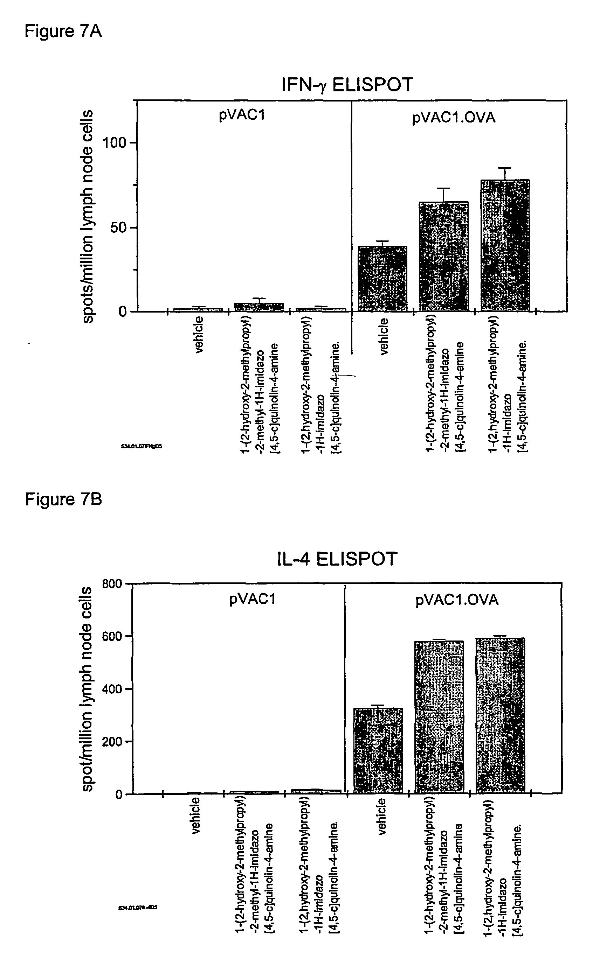

The effect on T cells 5 days after immunisation was investigated for the other two analogues given at the single dose of 30 mg/kg. Two fold increases in both IFN-γ and IL-4 producing CD4 T cells were induced by 1-(2-hydroxy-2-methylpropyl)-2-methyl-1H-imidazo[4,5-c]quinolin-4-amine. A similar effect was observed for 1-(2,hydroxy-2-methylpropyl)-1H-imidazo[4,5-c]quinolin-4-amine (Figure 7).

Overall, these results in this example show that the adjuvant effects are not restricted to imiquimod alone, but extend to the broader range of compounds in this chemical class.

### 7. Topical application is an effective route for administration of imiquimod with nucleic acid vaccination.

Cartridges were prepared using the WRG7077Gag/Nef plasmid, PMID immunisations and T cell responses were as described in example 5. Here, imiquimod was administered subcutaneously at a dose of 30 mg/kg. An additional group was included where imiquimod was administered by application of 20 µl of a 5% (w/v)cream formulation (ie. Aldara) applied topically at the PMID site immediately after immunisation. Spleens were collected for analysis 6 and 11 days after the boost immunisation.

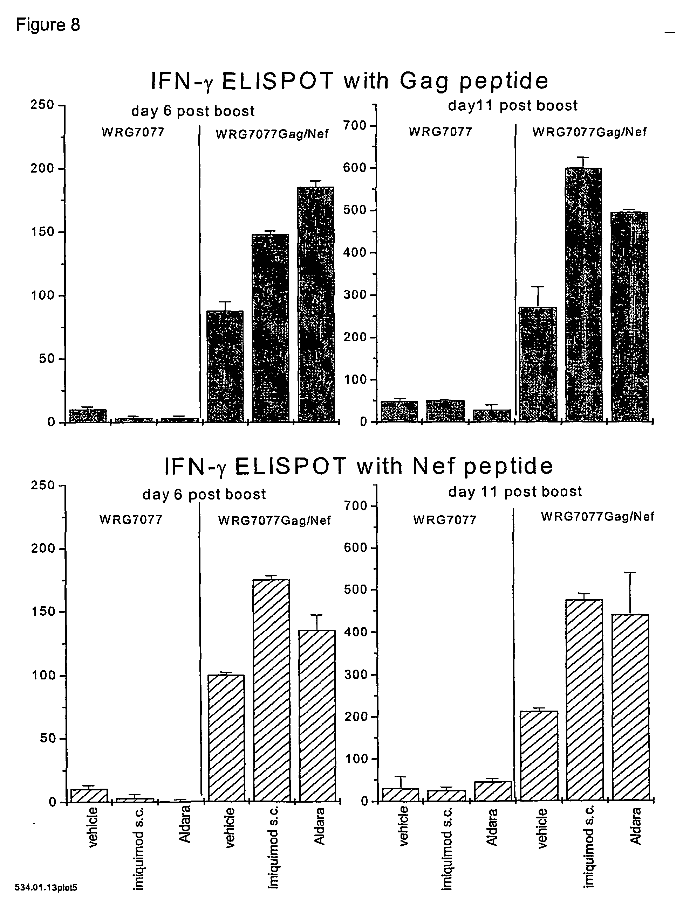

A substantial increase in cytotoxic T cells was observed when imiquimod was co-administered, either as s.c. injection or topically, compared with the group that received plasmid with s.c vehicle alone (ie. approximately 2 fold 11 days after boost) (Figure 8). These findings show that topically applied imiquimod is an effective adjuvant for PMID and suggest that subcutaneous injection is a relevant surrogate route for topical application in investigations of adjuvants combined with PMID.

### 8. Imiquimod is an effective adjuvant for the prevention of tumour growth.

Cartridges were prepared using the pVAC1.OVA plasmid (as described in example 2). Groups of 12 mice were immunised by PMID as described in example 5, except that cartridges contained 0.1 µg of DNA and imiquimod was administered subcutaneously at a dose of 30 mg/kg.

### Tumour challenge

Two weeks after the boost, EG7-OVA cells were implanted by needle injection subcutaneously in the flank of each mouse (100,000 cells/mouse) and tumour growth monitored. The E.G7-OVA cells were originally generated by F. Carbone (Moore, M.W., Carbone, F.R. and Bevan, M.J., Cell, 54, 777-785, 1988) by transfecting the mouse ascites lymphoma lymphoblast cell line, EL4, to stably express chicken ovalbumin.

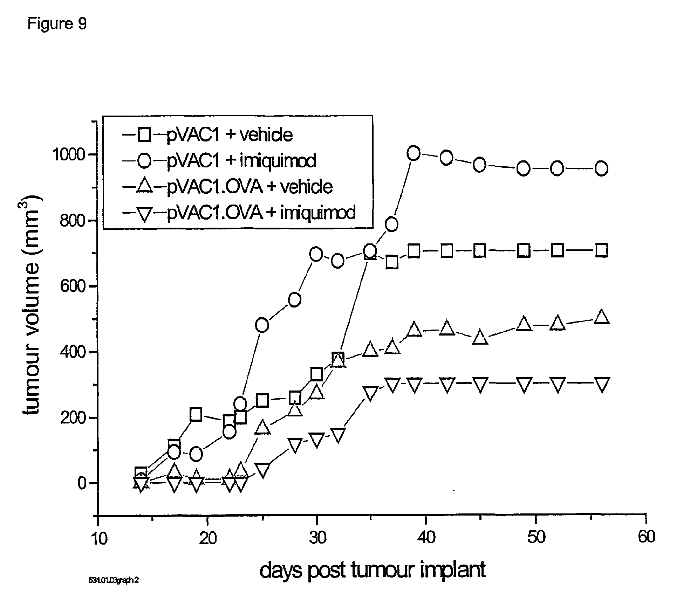

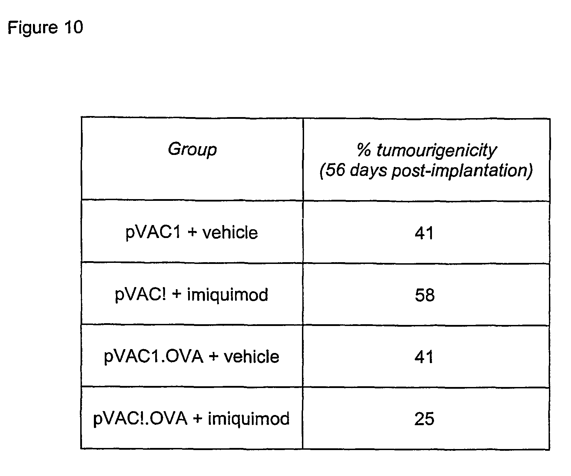

The appearance of palpable tumours was most delayed for mice immunised with pVAC1.OVA + imiquimod compared with pVAC1.OVA alone (Figure 9). Palpable tumours appeared earlier and grew most rapidly for control groups of mice (ie. mice immunised with empty vector ± imiquimod). Tumourigenicity was 25% for the mice immunised with pVAC1.OVA + imiquimod. In contrast, tumourigenicity was greater than 40% for all other groups(Figure 10). These results show that imiquimod can improve the antitumour effect of PMID, and suggest that imiquimod will be an effective adjuvant with nucleic acid for treatment of disease.

## Claims

1. A vaccine comprising (i) an immunogen component comprising a nucleotide sequence encoding an antigenic peptide or protein associated with a disease state, and (ii) an adjuvant component for enhancing an immune response to the antigenic peptide or protein encoded by the nucleotide sequence comprising, wherein the adjuvant component comprises a 1 H-imidazo[4,5-c]quinolin-4-amine derivative, and wherein the adjuvant component is administered between about 1 day prior to and about 3 days post administration of the immunogen component.

2. A vaccine according to claim 1 wherein the components are within a single pharmaceutically acceptable formulation.

3. A vaccine according to claim 1 or claim 2, wherein the 1 H-imidazo[4,5-c]quinolin-4-amine derivative is a compound defined by one of formulae I-VI: 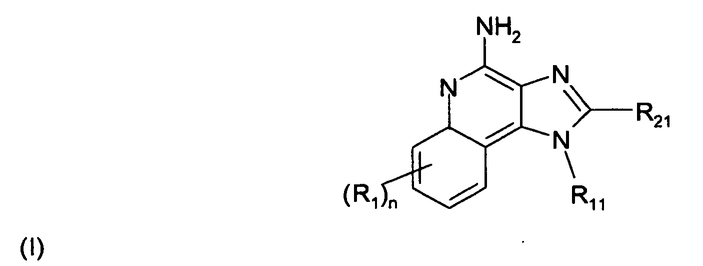 wherein
R₁₁ is selected from the group consisting of straight or branched chain alkyl, hydroxyalkyl, acyloxyalkyl, benzyl, (phenyl)ethyl and phenyl, said benzyl, (phenyl)ethyl or phenyl substituent being optionally substituted on the benzene ring by one or two moieties independently selected from the group consisting of alkyl of one to about four carbon atoms, alkoxy of one to about four carbon atoms and halogen, with the proviso that if said benzene ring is substituted by two of said moieties, then said moieties together contain no more than 6 carbon atoms; R₂₁ is selected from the group consisting of hydrogen, alkyl of one to about eight carbon atoms, benzyl, (phenyl)ethyl and phenyl, the benzyl, (phenyl)ethyl or phenyl substituent being optionally substituted on the benzene ring by one or two moieties independently selected from the group consisting of alkyl of one to about four carbon atoms, alkoxy of one to about four carbon atoms and halogen, with the proviso that when the benzene ring is substituted by two of said moieties, then the moieties together contain no more than 6 carbon atoms; and each R₁ is independently selected from the group consisting of hydrogen, alkoxy of one to about four carbon atoms, halogen and alkyl of one to about four carbon atoms, and n is an integer from 0 to 2, with the proviso that if n is 2, then said R₁₁ groups together contain no more than 6 carbon atoms; 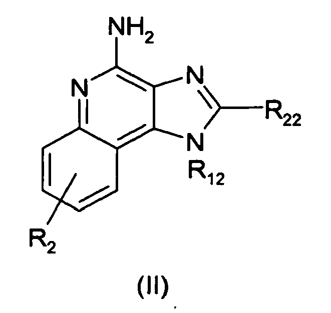 wherein
R₁₂ is selected from the group consisting of straight chain or branched chain alkenyl containing 2 to about 10 carbon atoms and substituted straight chain or branched chain alkenyl containing 2 to about 10 carbon atoms, wherein the substituent is selected from the group consisting of straight chain or branched chain alkyl containing 1 to about 4 carbon atoms and
cycloalkyl containing 3 to about 6 carbon atoms; and cycloalkyl containing 3 to about 6 carbon atoms substituted by straight chain or branched chain alkyl containing 1 to about 4 carbon atoms; and R₂₂ is selected from the group consisting of hydrogen, straight chain or branched chain alkyl containing one to about eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl containing one to about four carbon atoms, straight chain or branched chain alkoxy containing one to about four carbon atoms, and halogen, with the proviso that when the benzene ring is substituted by two such moieties, then the moieties together contain no more than 6 carbon atoms; and each R₂ is independently selected from the group consisting of straight chain or branched chain alkoxy containing one to about four carbon atoms, halogen, and straight chain or branched chain alkyl containing one to about four carbon atoms, and n is an integer from zero to 2, with the proviso that if n is 2, then said R₂ groups together contain no more than 6 carbon atoms; 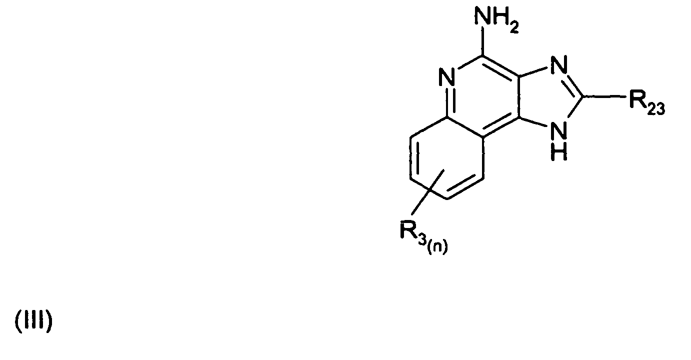 wherein
R₂₃ is selected from the group consisting of hydrogen, straight chain or branched chain alkyl of one to about eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl of one to about four carbon atoms, straight chain or branched chain alkoxy of one to about four carbon atoms, and halogen, with the proviso that when the benzene ring is substituted by two such moieties, then the moieties together-contain no more than 6 carbon atoms; and each R₅ is independently selected from the group consisting of straight chain or branched chain alkoxy of one to about four-carbon atoms, halogen, and 30 straight chain or branched chain alkyl of one to about four carbon atoms, and n is an integer from zero to 2, with the proviso that if n is 2, then said R₃ groups together contain no more than 6 carbon atoms; 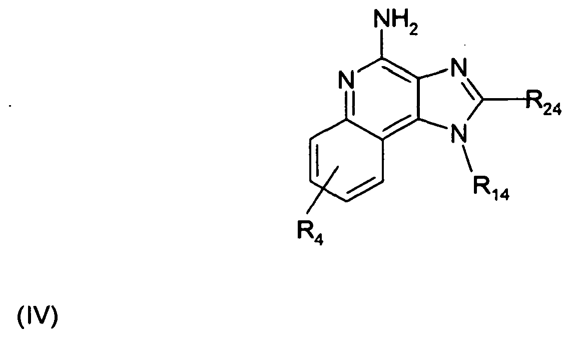 wherein
R₁₄ is -CHR_{A}R_{B} wherein R_{B} is hydrogen or a carbon-carbon bond, with the proviso that when R_{B} is hydrogen R_{A} is alkoxy of one to about four carbon atoms, hydroxyalkoxy of one to about four carbon atoms, 1-alkynyl of two to about ten carbon atoms, tetrahydropyranyl, alkoxyalkyl wherein the alkoxy moiety contains one to about four carbon atoms and the alkyl moiety contains one to about four carbon atoms, 2-, 3-, or 4-pyridyl, and with the further proviso that when R_{B} is a carbon-carbon bond R_{B} and R_{A} together form a tetrahydrofuranyl group optionally substituted with one or more substituents independently selected from the group consisting of hydroxy and hydroxyalkyl of one to about four carbon atoms; R₂₄ is selected from the group consisting of hydrogen, alkyl of one to about four carbon atoms, phenyl, and substituted phenyl wherein the substituent is selected from the group consisting of alkyl of one to about four carbon atoms, alkoxy of one to about four carbon atoms, and halogen; and R₄ is selected from the group consisting of hydrogen, straight chain or branched chain alkoxy containing one to about four carbon atoms, halogen, and straight chain or branched chain alkyl containing one to about four carbon atoms; 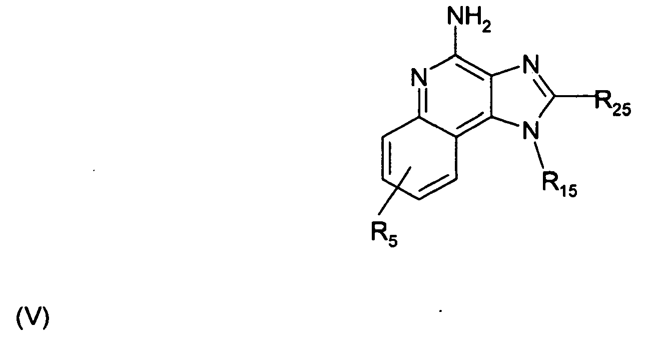 wherein
R₁₅ is selected from the group consisting of: hydrogen; straight chain or branched chain alkyl containing one to about ten carbon atoms and substituted straight chain or branched chain alkyl containing one to about ten carbon atoms, wherein the substituent is selected from the group consisting of cycloalkyl containing three to about six carbon atoms and cycloalkyl containing three to about six carbon atoms substituted by straight chain or branched chain alkyl containing one to about four carbon atoms; straight chain or branched chain alkenyl containing two to about ten carbon atoms and substituted straight chain or branched chain alkenyl containing two to about ten carbon atoms, wherein the substituent is selected from the group consisting of cycloalkyl containing three to about six carbon atoms and cycloalkyl containing three to about six carbon atoms substituted by straight chain or branched chain alkyl containing one to about four carbon atoms; hydroxyalkyl of one to about six carbon atoms; alkoxyalkyl wherein the alkoxy moiety contains one to about four carbon atoms and the alkyl moiety contains one to about six carbon atoms; acyloxyalkyl wherein the acyloxy moiety is alkanoyloxy of two to about four carbon atoms or benzoyloxy, and the alkyl moiety contains one to about six carbon atoms; benzyl; (phenyl)ethyl; and phenyl; said benzyl, (phenyl)ethyl or phenyl substituent being optionally substituted on the benzene ring by one or two moieties independently selected from the group consisting of
alkyl of one to about four carbon atoms, alkoxy of one to about four carbon atoms, and halogen, with the proviso that when said benzene ring is substituted by two of said moieties, then the moieties together contain no more than six carbon atoms;
R₂₅ is 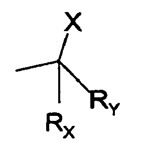 wherein
R_{X} and R_{Y} are independently selected from the group consisting of hydrogen, alkyl of one to about four carbon atoms, phenyl, and substituted phenyl wherein the substituent is elected from the group consisting of alkyl of one to about four carbon atoms, alkoxy of one to about four carbon atoms, and halogen; X is selected from the group consisting of alkoxy containing one to about four carbon atoms, alkoxyalkyl wherein the alkoxy moiety contains one to about four carbon atoms and the alkyl moiety contains one to about four carbon atoms, haloalkyl of one to about four carbon atoms, alkylamido wherein the alkyl group contains one to about four carbon atoms, amino, substituted amino wherein the substituent is alkyl or hydroxyalkyl of one to about four carbon atoms, azido, alkylthio of one to about four carbon atoms; and R₅ is selected from the group consisting of hydrogen, straight chain or branched chain alkoxy containing one to about four carbon atoms, halogen, and straight chain or branched chain alkyl containing one to about four carbon atoms; 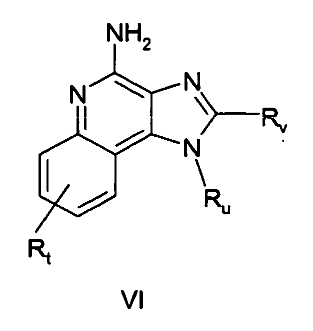 Wherein
Rₜ is selected from the group consisting of hydrogen, straight chain or branched chain alkoxy containing one to about four carbon atoms, halogen, and straight chain or branched chain alkyl containing one to about four carbon atoms;
Rᵤ is 2-methylpropyl or 2-hydroxy-2-methylpropyl; and
Rᵥ is hydrogen, alkyl of one to about six carbon atoms, or alkoxyalkyl wherein the alkoxy moiety contains one to about four carbon atoms and the alkyl moiety contains one to about four carbon atoms.
or a pharmaceutically acceptable salt of any of the foregoing.

4. A vaccine according to claim 3 wherein the 1 H-imidazo[4,5-c]quinolin-4-amine derivative is a compound of formula VI.

5. A vaccine according to claim 4 wherein Rₜ is hydrogen.

6. A vaccine according to claim 5 wherein Rᵤ is 2-methylpropyl or 2-hydroxy-2-methylpropyl, and Rᵥ is hydrogen, methyl, or ethoxymethyl.

7. A vaccine according to claim 6 wherein the compound is selected from the group consisting of
1-(2-methylpropyl)-1H-imidazo[4,5-c]quinolin-4-amine
1-(2-hydroxy-2-methylpropyl)-1H-imidazo[4,5-c]quinolin-4-amine
1-(2-hydrozy-2-methylpropyl)-2-methyl-1H-imidazo[4,5-c]quinolin-4-amine
1-(2-hydroxy-2-methylpropyl)-2-ethoxymethyl-1H-imidazo[4,5-c]quinoline-4-amine.

8. A vaccine according to any preceding claim wherein each component is in a form suitable for administration via any of the oral, nasal, topical, pulmonary, intramuscular, subcutaneous or intradermal routes.

9. A vaccine according to claim 8 wherein the immunogen component is in a form suitable for administration using a particle mediated gene transfer technique.

10. A vaccine according to claim 9 wherein the adjuvant component is in a form suitable for administration using a particle mediated gene transfer technique.

11. Use of a 1H-imidazo[4,5-c]quinolin-4-amine derivative in the manufacture of a medicament for enhancing immune responses initiated by an antigenic peptide in the treatment of a mammal in a disease state selected from viral, bacterial or parasitic infection, cancer, allergy or autoimmune disorder, said peptide being expressed as a result of administration to a mammal of a nucleotide sequence encoding for said peptide, and wherein the 1H-imidazo[4,5-c]quinolin-4-amine derivative is administered between about 1 day prior to and about 3 days post administration of the nucleotide sequence encoding for said peptide.

12. The use according to claim 11 wherein the 1H-imidazo[4,5-c]quinolin-4-amine derivative is as defined in claim 3.

13. The use according to claim 12 wherein the 1H-imidazo[4,5-c]quinolin-4-amine derivative is as defined in claim 4.

14. The use according to any one of claims 11 to 13 wherein the 1H-imidazo[4,5-c]quinolin-4-amine derivative is administered at a dose of between about 1 mg/kg to 50 mg/kg

15. The use according to any one of claims 11 to 14 wherein the nucleotide sequence encoding for said peptide is administered by a particle mediated gene transfer technique and the 1 H-imidazo[4,5-c]quinolin-4-amine derivative is administered topically at the site of administration of the nucleotide sequence.

16. A 1H-imidazo[4,5-c]quinolin-4-amine derivative for use as an adjuvant to enhance an immune response initiated by the antigenic peptide, said peptide being expressed as a result of administration to a mammal of a nucleotide sequence encoding for said peptide, wherein the 1H-imidazo[4,5-c]quinolin-4-amine derivative is administered between about 1 day prior to and about 3 days post administration of the nucleotide sequence encoding for said peptide.

17. A 1H-imidazo[4,5-c]quinolin-4-amine derivative according to claim 16 wherein the 1H-imidazo[4,5-c]quinolin-4-amine derivative is as defined in claim 3.

18. A 1H-imidazo[4,5-c]quinolin-4-amine derivative according to claim 17 wherein the 1H-imidazo[4,5-c]quinolin-4-amine derivative is as defined in claim 4.

## Patentansprüche

1. Impfstoff, der (i) eine Immunogenkomponente, die eine Nukleotidsequenz umfaßt, die ein antigenes Peptid oder Protein codiert, das mit einem Krankheitszustand assoziiert ist, und (ii) eine Adjuvanskomponente zur Steigerung einer Immunreaktion auf das antigene Peptid oder Protein, das durch die Nukleotidsequenz codiert wird, umfaßt, worin die Adjuvanskomponente ein 1H-Imidazo[4,5-c]chinolin-4-amin-Derivat umfaßt, und worin die Adjuvanskomponente zwischen etwa 1 Tag vor und etwa 3 Tagen nach Verabreichung der Immunogenkomponente verabreicht wird.

2. Impfstoff gemäß Anspruch 1, worin die Komponenten in einer einzelnen pharmazeutisch annehmbaren Formulierung vorliegen.

3. Impfstoff gemäß Anspruch 1 oder Anspruch 2, worin das 1H-Imidazo[4,5-c]chinolin-4-amin-Derivat eine Verbindung ist, die durch eine der Formeln (I) bis (IV) definiert ist: 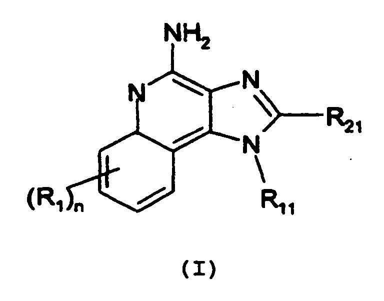 worin
R₁₁ aus der Gruppe ausgewählt ist, die aus geradkettigem oder verzweigtkettigem Alkyl, Hydroxyalkyl, Acyloxyalkyl, Benzyl, (Phenyl)ethyl und Phenyl besteht, wobei der Benzyl-, (Phenyl)ethyl- oder Phenyl-Substituent gegebenenfalls auf dem Benzol-Ring mit einem oder zwei Resten substituiert ist, die unabhängig aus der Gruppe ausgewählt sind, die aus Alkyl von einem bis etwa vier Kohlenstoffatomen, Alkoxy von einem bis etwa vier Kohlenstoffatomen und Halogen besteht, mit der Maßgabe, daß, falls der Benzol-Ring mit zwei der Reste substituiert ist, dann enthalten die Reste zusammen nicht mehr als 6 Kohlenstoffatome; R₂₁ aus der Gruppe ausgewählt ist, die aus Wasserstoff, Alkyl von einem bis etwa acht Kohlenstoffatomen, Benzyl, (Phenyl)ethyl und Phenyl besteht, wobei der Benzyl-, (Phenyl)ethyl- oder Phenyl-Substituent gegebenenfalls auf dem Benzol-Ring mit einem oder zwei Resten substituiert ist, die unabhängig aus der Gruppe ausgewählt sind, die aus Alkyl von einem bis etwa vier Kohlenstoffatomen, Alkoxy von einem bis etwa vier Kohlenstoffatomen und Halogen besteht, mit der Maßgabe, daß, wenn der Benzol-Ring mit zwei der Reste substituiert ist, dann enthalten die Reste zusammen nicht mehr als 6 Kohlenstoffatome; und jedes R₁ unabhängig aus der Gruppe ausgewählt ist, die aus Hydroxy, Alkoxy von einem bis etwa vier Kohlenstoffatomen, Halogen und Alkyl von einem bis etwa vier Kohlenstoffatomen besteht, und n eine ganze Zahl von 0 bis 2 ist, mit der Maßgabe, daß, falls n 2 ist, dann enthalten die R₁₁-Gruppen zusammen nicht mehr als 6 Kohlenstoffatome; 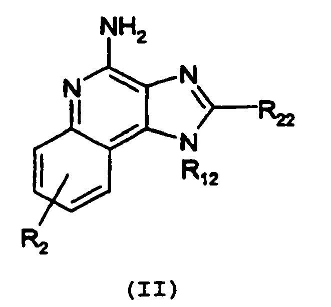 worin
R₁₂ aus der Gruppe ausgewählt ist, die aus geradkettigem oder verzweigtkettigem Alkenyl, das 2 bis etwa 10 Kohlenstoffatome enthält, und substituiertem geradkettigem oder verzweigtkettigem Alkenyl, das 2 bis etwa 10 Kohlenstoffatome enthält, besteht, worin der Substituent aus der Gruppe ausgewählt ist, die aus geradkettigem oder verzweigtkettigem Alkyl, das 1 bis etwa 4 Kohlenstoffatome enthält, und Cycloalkyl, das 3 bis etwa 6 Kohlenstoffatome enthält; und Cycloalkyl, das 3 bis etwa 6 Kohlenstoffatome enthält, substituiert mit geradkettigem oder verzweigtkettigem Alkyl, das ein bis etwa vier Kohlenstoffatome enthält, besteht; und R₂₂ aus der Gruppe ausgewählt ist, die aus Wasserstoff, geradkettigem oder verzweigtkettigem Alkyl, das 1 bis etwa 8 Kohlenstoffatome enthält, Benzyl, (Phenyl)ethyl und Phenyl besteht, wobei der Benzyl-, (Phenyl)ethyl- oder Phenyl-Substituent gegebenenfalls auf dem Benzol-Ring mit einem oder zwei Resten substituiert sind, die unabhängig aus der Gruppe ausgewählt sind, die aus geradkettigem oder verzweigtkettigem Alkyl, das ein bis etwa vier Kohlenstoffatome enthält, geradkettigem oder verzweigtkettigem Alkoxy, das ein bis etwa vier Kohlenstoffatome enthält, und Halogen besteht, mit der Maßgabe, daß, wenn der Benzol-Ring mit zwei solcher Reste substituiert ist, dann enthalten die Reste zusammen nicht mehr als 6 Kohlenstoffatome; und jedes R₂ unabhängig aus der Gruppe ausgewählt ist, die aus geradkettigem oder verzweigtkettigem Alkoxy, das ein bis etwa vier Kohlenstoffatome enthält, Halogen und geradkettigem oder verzweigtkettigem Alkyl, das ein bis etwa vier Kohlenstoffatome enthält, besteht und n eine ganze Zahl von Null bis 2 ist, mit der Maßgabe, daß, falls n 2 ist, dann enthalten die R₂-Gruppen zusammen nicht mehr als 6 Kohlenstoffatome; 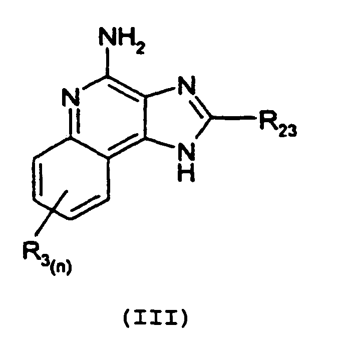 worin
R₂₃ aus der Gruppe ausgewählt ist, die aus Wasserstoff, geradkettigem oder verzweigtkettigem Alkyl von einem bis etwa acht Kohlenstoffatomen, Benzyl, (Phenyl)ethyl und Phenyl besteht, wobei der Benzyl-, (Phenyl)ethyl- oder Phenyl-Substituent gegebenenfalls auf dem Benzol-Ring mit einem oder zwei Resten substituiert ist, die unabhängig aus der Gruppe ausgewählt sind, die aus geradkettigem oder verzweigtkettigem Alkyl von einem bis etwa vier Kohlenstoffatomen, geradkettigem oder verzweigtkettigem Alkoxy von einem bis etwa vier Kohlenstoffatomen und Halogen besteht, mit der Maßgabe, daß, wenn der Benzol-Ring mit zwei solcher Reste substituiert ist, dann enthalten die Reste zusammen nicht mehr als 6 Kohlenstoffatome; und jedes R₅ unabhängig aus der Gruppe ausgewählt ist, die aus geradkettigem oder verzweigtkettigem Alkoxy von einem bis etwa vier Kohlenstoffatomen, Halogen und 30 geradkettigem oder verzweigtkettigem Alkyl von einem bis etwa vier Kohlenstoffatomen besteht, und n eine ganze Zahl von Null bis 2 ist, mit der Maßgabe, daß, falls n 2 ist, dann enthalten die R₃-Gruppen zusammen nicht mehr als 6 Kohlenstoffatome; 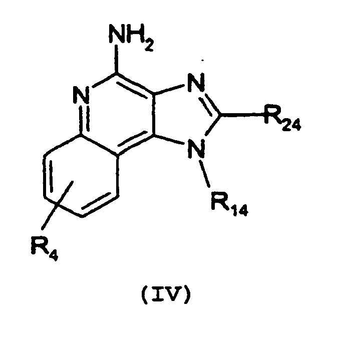 worin
R₁₄ -CHR_{A}R_{B} ist, worin R_{B} Wasserstoff oder eine Kohlenstoff-Kohlenstoff-Bindung ist, mit der Maßgabe, daß, wenn R_{B} Wasserstoff ist, dann ist R_{A} Alkoxy von einem bis etwa vier Kohlenstoffatomen, Hydroxyalkoxy von einem bis etwa vier Kohlenstoffatomen, 1-Alkinyl von zwei bis etwa zehn Kohlenstoffatomen, Tetrahydropyranyl, Alkoxyalkyl, worin der Alkoxy-Rest ein bis vier Kohlenstoffatome enthält und der Alkyl-Rest ein bis etwa vier Kohlenstoffatome enthält, 2-, 3- oder 4-Pyridyl, und mit der weiteren Maßgabe, daß, wenn R_{B} eine Kohlenstoff-Kohlenstoff-Bindung ist, R_{B} und R_{A} zusammen eine Tetrahydrofuranyl-Gruppe bilden, die gegebenenfalls mit einem oder mehreren Substituenten substituiert ist, die unabhängig aus der Gruppe ausgewählt sind, die aus Hydroxy und Hydroxyalkyl von einem bis etwa vier Kohlenstoffatomen besteht; R₂₄ aus der Gruppe ausgewählt ist, die aus Wasserstoff, Alkyl von einem bis etwa vier Kohlenstoffatomen, Phenyl und substituiertem Phenyl besteht, worin der Substituent aus der Gruppe ausgewählt ist, die aus Alkyl von einem bis etwa vier Kohlenstoffatomen, Alkoxy von einem bis etwa vier Kohlenstoffatomen und Halogen besteht; und R₄ aus der Gruppe ausgewählt ist, die aus Wasserstoff, geradkettigem oder verzweigtkettigem Alkoxy, das ein bis etwa vier Kohlenstoffatome enthält, Halogen und geradkettigem oder verzweigtkettigem Alkyl, das ein bis etwa vier Kohlenstoffatome enthält, besteht; 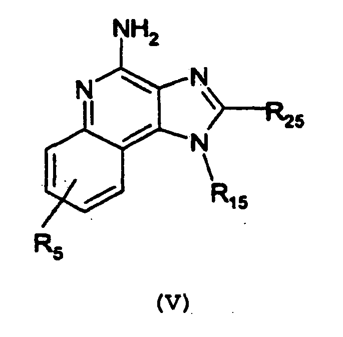 worin
R₁₅ aus der Gruppe ausgewählt ist, bestehend aus: Wasserstoff; geradkettigem oder verzweigtkettigem Alkyl, das ein bis etwa zehn Kohlenstoffatome enthält, und substituiertem geradkettigem oder verzweigtkettigem Alkyl, das ein bis etwa zehn Kohlenstoffatome enthält, worin der Substituent aus der Gruppe ausgewählt ist, die aus Cycloalkyl, das drei bis etwa sechs Kohlenstoffatome enthält, und Cycloalkyl, das drei bis etwa sechs Kohlenstoffatome enthält, substituiert mit geradkettigem oder verzweigtkettigem Alkyl, das ein bis etwa vier Kohlenstoffatome enthält; geradkettigem oder verzweigtkettigem Alkenyl, das zwei bis etwa zehn Kohlenstoffatome enthält, und substituiertem geradkettigem oder verzweigtkettigem Alkenyl, das zwei bis etwa zehn Kohlenstoffatome enthält, besteht, worin der Substituent aus der Gruppe ausgewählt ist, die aus Cycloalkyl, das drei bis etwa sechs Kohlenstoffatome enthält, und Cycloalkyl, das drei bis etwa sechs Kohlenstoffatome enthält, substituiert mit geradkettigem oder verzweigtkettigem Alkyl, das ein bis etwa vier Kohlenstoffatome enthält; Hydroxyalkyl von ein bis etwa sechs Kohlenstoffatomen; Alkoxyalkyl, worin der Alkoxy-Rest ein bis etwa vier Kohlenstoffatome enthält und der Alkyl-Rest ein bis etwa sechs Kohlenstoffatome enthält; Acyloxyalkyl, worin der Acyloxy-Rest Alkanoyloxy von zwei bis etwa vier Kohlenstoffatomen oder Benzyloxy ist, und der Alkyl-Rest ein bis etwa sechs Kohlenstoffatome enthält; Benzyl; (Phenyl)ethyl; und Phenyl besteht; wobei der Benzyl-, (Phenyl)ethyl- oder Phenyl-Substituent gegebenenfalls auf dem Benzol-Ring mit einem oder zwei Resten substituiert ist, die unabhängig aus der Gruppe ausgewählt sind, die aus Alkyl von einem bis etwa vier Kohlenstoffatomen, Alkoxy von einem bis etwa vier Kohlenstoffatomen und Halogen besteht, mit der Maßgabe, daß, wenn der Benzol-Ring mit zwei dieser Reste substituiert ist, dann enthalten die Reste zusammen nicht mehr als sechs Kohlenstoffatome;
R₂₅ ist 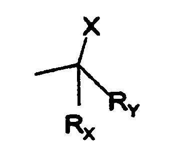 worin
R_{X} und R_{Y} unabhängig aus der Gruppe ausgewählt sind, die aus Wasserstoff, Alkyl von einem bis etwa vier Kohlenstoffatomen, Phenyl und substituiertem Phenyl besteht, worin der Substituent aus der Gruppe ausgewählt ist, die aus Alkyl von einem bis etwa vier Kohlenstoffatomen, Alkoxy von ein bis etwa vier Kohlenstoffatomen und Halogen besteht; X aus der Gruppe ausgewählt ist, die aus Alkoxy, das ein bis etwa vier Kohlenstoffatome enthält, Alkoxyalkyl, worin der Alkoxy-Rest ein bis etwa vier Kohlenstoffatome enthält und der Alkyl-Rest ein bis etwa vier Kohlenstoffatome enthält, Halogenalkyl von einem bis etwa vier Kohlenstoffatomen, Alkylamido, worin die Alkyl-Gruppe ein bis etwa vier Kohlenstoffatome enthält, Amino, substituiertem Amino, worin der Substituent Alkyl oder Hydroxyalkyl von einem bis etwa vier Kohlenstoffatomen ist, Azido, Alkylthio von einem bis etwa vier Kohlenstoffatomen besteht; und R₅ aus der Gruppe ausgewählt ist, die aus Wasserstoff, geradkettigem oder verzweigtkettigem Alkoxy, das ein bis etwa vier Kohlenstoffatome enthält, Halogen, und geradkettigem und verzweigtkettigem Alkyl, das ein bis etwa vier Kohlenstoffatome enthält, besteht; 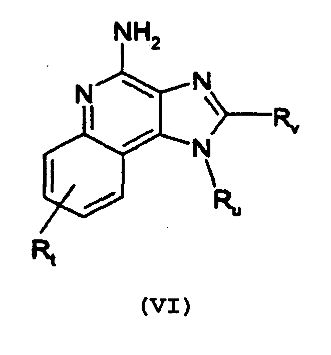 worin
Rₜ aus der Gruppe ausgewählt ist, die aus Wasserstoff, geradkettigem oder verzweigtkettigem Alkoxy, das ein bis etwa vier Kohlenstoffatome enthält, Halogen und geradkettigem oder verzweigtkettigem Alkyl, das ein bis etwa vier Kohlenstoffatome enthält, besteht;
Rᵤ 2-Methylpropyl oder 2-Hydroxy-2-methylpropyl ist; und
Rᵥ Wasserstoff, Alkyl von einem bis etwa sechs Kohlenstoffatomen oder Alkoxyalkyl ist, worin der Alkoxy-Rest ein bis etwa vier Kohlenstoffatome enthält und der Alkyl-Rest ein bis etwa vier Kohlenstoffatome enthält;
oder ein pharmazeutisch annehmbares Salz jedes der vorangegangenen.

4. Impfstoff gemäß Anspruch 3, worin das 1H-Imidazo[4,5-c]chinolin-4-amin-Derivat eine Verbindung der Formel (VI) ist.

5. Impfstoff gemäß Anspruch 4, worin Rₜ Wasserstoff ist.

6. Impfstoff gemäß Anspruch 5, worin Rᵤ 2-Methylpropyl oder 2-Hydroxy-2-methylpropyl ist und Rᵥ Wasserstoff, Methyl oder Ethoxymethyl ist.

7. Impfstoff gemäß Anspruch 6, worin die Verbindung aus der Gruppe ausgewählt ist, die aus
1-(2-Methylpropyl)-1H-imidazo[4,5-c]chinolin-4-amin,
1-(2-Hydroxy-2-methylpropyl)-1H-imidazo[4,5-c]chinolin-4-amin,
1-(2-Hydroxy-2-methylpropyl)-2-methyl-1H-imidazo[4,5-c]chinolin-4-amin,
1-(2-Hydroxy-2-methylpropyl)-2-ethoxymethyl-1H-imidazo[4,5-c]chinolin-4-amin
besteht.

8. Impfstoff gemäß einem der vorangegangenen Ansprüche, worin jede Komponente in einer Form ist, die für die Verabreichung über jeden der oralen, nasalen, topischen, pulmonaren, intramuskulären, subkutanen oder intradermalen Wege geeignet ist.

9. Impfstoff gemäß Anspruch 8, worin die Immunogenkomponente in einer Form ist, die für die Verabreichung unter Verwendung einer Partikelvermittelten Gentransfertechnik geeignet ist.

10. Impfstoff gemäß Anspruch 9, worin die Adjuvanskomponente in einer Form ist, die für die Verabreichung unter Verwendung einer Partikelvermittelten Gentransfertechnik geeignet ist.

11. Verwendung eines 1H-Imidazo[4,5-c]chinolin-4-amin-Derivats in der Herstellung eines Medikaments zur Steigerung von Immunreaktionen, die durch ein antigenes Peptid erzeugt wird, in der Behandlung eines Säugetiers in einem Krankheitszustand, ausgewählt aus viraler, bakterieller oder parasitärer Infektion, Krebs, Allergie oder Autoimmunstörung, wobei das Peptid als ein Ergebnis der Verabreichung einer Nukleotidsequenz, die für das Peptid codiert, an ein Säugetier exprimiert wird, und worin das 1H-Imidazo[4,5-c]chinolin-4-amin-Derivat zwischen etwa 1 Tag vor und etwa 3 Tagen nach Verabreichung der Nukleotidsequenz, die für das Peptid codiert, verabreicht wird.

12. Verwendung gemäß Anspruch 11, worin das 1H-Imidazo[4,5-c]chinolin-4-amin-Derivat eins wie in Anspruch 3 definiert ist.

13. Verwendung gemäß Anspruch 12, worin das 1H-Imidazo[4,5-c]chinolin-4-amin-Derivat eins wie in Anspruch 4 definiert ist.

14. Verwendung gemäß einem der Ansprüche 11 bis 13, worin das 1H-Imidazo[4,5-c]chinolin-4-amin-Derivat bei einer Dosis von zwischen etwa 1 mg/kg bis 50 mg/kg verabreicht wird.

15. Verwendung gemäß einem der Ansprüche 11 bis 14, worin die Nukleotidsequenz, die für das Peptid codiert, durch eine Partikel-vermittelte Gentransfertechnik verabreicht wird, und das 1H-Imidazo[4,5-c]chinolin-4-amin-Derivat topisch an der Verabreichungsstelle der Nukleotidsequenz verabreicht wird.

16. 1H-Imidazo[4,5-c]chinolin-4-amin-Derivat für die Verwendung als ein Adjuvans zum Steigern einer Immunreaktion, die durch das antigene Peptid hervorgerufen wird, wobei das Peptid als ein Ergebnis der Verabreichung einer Nukleotidsequenz, die für das Peptid codiert, an ein Säugetier exprimiert wird, worin das 1H-Imidazo[4,5-c]chinolin-4-amin-Derivat zwischen etwa 1 Tag vor und etwa 3 Tagen nach Verabreichung der Nukleotidsequenz, die für das Peptid codiert, verabreicht wird.

17. 1H-Imidazo[4,5-c]chinolin-4-amin-Derivat gemäß Anspruch 16, worin das 1H-Imidazo[4,5-c]chinolin-4-amin-Derivat eins wie in Anspruch 3 definiert ist.

18. 1H-Imidazo[4,5-c]chinolin-4-amin-Derivat gemäß Anspruch 17, worin das 1H-Imidazo[4,5-c]chinolin-4-amin-Derivat eins wie in Anspruch 4 definiert ist.

## Revendications

1. Vaccin comprenant (i) un composant immunogène comprenant une séquence de nucléotides codant pour un peptide antigénique ou une protéine antigénique associé(e) à un état pathologique, et (ii) un composant adjuvant destiné à améliorer une réponse immunitaire au peptide antigénique ou à la protéine antigénique codé(e) par la séquence de nucléotides, dans lequel le composant adjuvant comprend un dérivé de 1H-imidazo[4,5-c]quinolin-4-amine, et dans lequel le composant adjuvant est administré entre environ 1 jour avant et environ 3 jours après l'administration du composant immunogène.

2. Vaccin selon la revendication 1, dans lequel les composants se trouvent à l'intérieur d'une formulation pharmaceutiquement acceptable unique.

3. Vaccin selon la revendication 1 ou la revendication 2, dans lequel le dérivé de 1H-imidazo[4,5-c]quinolin-4-amine est un composé défini par l'une des formules I à VI : 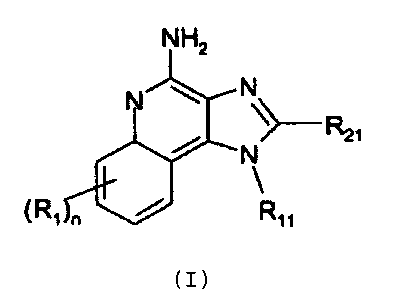 dans laquelle :
R₁₁ est choisi dans le groupe constitué par un groupe alkyle à chaîne linéaire ou ramifiée, un groupe hydroxyalkyle, acyloxyalkyle, benzyle, (phényl)éthyle et phényle, ledit substituant benzyle, (phényl)éthyle ou phényle étant facultativement substitué sur le cycle benzène par une ou deux fractions indépendamment choisies dans le groupe constitué par un groupe alkyle ayant de un à environ quatre atomes de carbone, un groupe alcoxy ayant de un à environ quatre atomes de carbone et un atome d'halogène, à condition que si ledit cycle benzène est substitué par deux desdites fractions, alors lesdites fractions ensemble ne contiennent pas plus de 6 atomes de carbone ; R₂₁ est choisi dans le groupe constitué par un atome d'hydrogène, un groupe alkyle ayant de un à environ huit atomes de carbone, un groupe benzyle, (phényl)éthyle et phényle, le substituant benzyle, (phényl)éthyle ou phényle étant facultativement substitué sur le cycle benzène par une ou deux fractions indépendamment choisies dans le groupe constitué par un groupe alkyle ayant de un à environ quatre atomes de carbone, un groupe alcoxy ayant de un à environ quatre atomes de carbone et un atome d'halogène, à condition que lorsque le cycle benzène est substitué par deux desdites fractions, alors les fractions ensemble ne contiennent pas plus de 6 atomes de carbone ; et chaque R₁ est indépendamment choisi dans le groupe constitué par un atome d'hydrogène, un groupe alcoxy ayant de un à environ quatre atomes de carbone, un atome d'halogène et un groupe alkyle ayant de un à environ quatre atomes de carbone, et n est un nombre entier de 0 à 2, à condition que si n vaut 2, alors lesdits groupes R₁₁ ensemble ne contiennent pas plus de 6 atomes de carbone ; 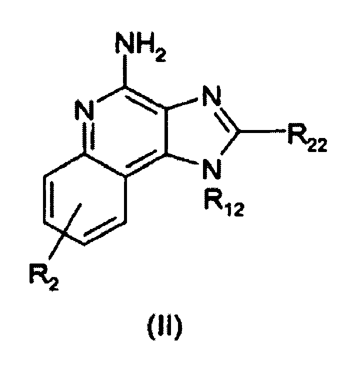
dans laquelle
R₁₂ est choisi dans le groupe constitué par un groupe alcényle à chaîne linéaire ou à chaîne ramifiée contenant de 2 à environ 10 atomes de carbone et un groupe alcényle à chaîne linéaire ou ramifié, substitué, et contenant de 2 à environ 10 atomes de carbone, dans lequel le substituant est choisi dans le groupe constitué par un groupe alkyle à chaîne linéaire ou à chaîne ramifiée contenant de 1 à environ 4 atomes de carbone et un groupe cycloalkyle contenant de 3 à environ 6 atomes de carbone ; et un groupe cycloalkyle contenant de 3 à environ 6 atomes de carbone substitués par un groupe alkyle à chaîne linéaire ou à chaîne ramifiée contenant de 1 à environ 4 atomes de carbone ; et R₂₂ est choisi dans le groupe constitué par un atome d'hydrogène, un groupe alkyle à chaîne linéaire ou ramifiée contenant de un à environ huit atomes de carbone, un groupe benzyle, (phényl)éthyle et phényle, le substituant benzyle, (phényl)éthyle ou phényle étant facultativement substitué sur le cycle benzène par une ou deux fractions indépendamment choisies dans le groupe constitué par un groupe alkyle à chaîne linéaire ou à chaîne ramifiée contenant de un à environ quatre atomes de carbone, un groupe alcoxy à chaîne linéaire ou ramifiée contenant de un à environ quatre atomes de carbone, et un atome d'halogène, à condition que lorsque le cycle benzène est substitué par deux de ces fractions, alors les fractions ensemble ne contiennent pas plus de 6 atomes de carbone ; et chaque R₂ est indépendamment choisi dans le groupe constitué par un groupe alcoxy à chaîne linéaire ou ramifiée contenant de un à environ quatre atomes de carbone, un atome d'halogène, et un groupe alkyle à chaîne linéaire ou ramifiée contenant de un à environ quatre atomes de carbone, et n est un nombre entier de zéro à 2, à condition que si n vaut 2, alors lesdits groupes R₂ ensemble ne contiennent pas plus de 6 atomes de carbone ; 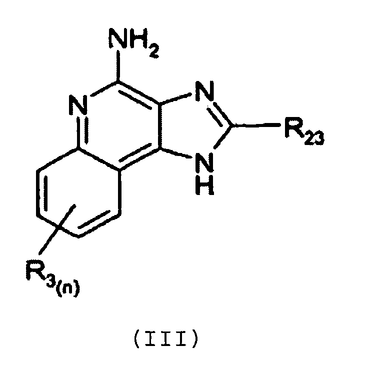 dans laquelle
R₂₃ est choisi dans le groupe constitué par un atome d'hydrogène, un groupe alkyle à chaîne linéaire ou ramifiée contenant de un à environ huit atomes de carbone, un groupe benzyle, (phényl)éthyle et phényle, le substituant benzyle, (phényl)éthyle ou phényle étant facultativement substitué sur le cycle benzène par une ou deux fractions indépendamment choisies dans le groupe constitué par un groupe alkyle à chaîne linéaire ou à chaîne ramifiée contenant de un à environ quatre atomes de carbone, un groupe alcoxy à chaîne linéaire ou ramifiée contenant de un à environ quatre atomes de carbone, et un atome d'halogène, à condition que lorsque le cycle benzène est substitué par deux de ces fractions, alors les fractions ensemble ne contiennent pas plus de 6 atomes de carbone ; et chaque R₅ est indépendamment choisi dans le groupe constitué par un groupe alcoxy à chaîne linéaire ou ramifiée contenant de un à environ quatre atomes de carbone, un atome d'halogène, et des groupes alkyle à chaîne linéaire ou ramifiée contenant de un à environ quatre atomes de carbone, et n est un nombre entier de zéro à 2, à condition que si n vaut 2, alors lesdits groupes R₃ ensemble ne contiennent pas plus de 6 atomes de carbone ; 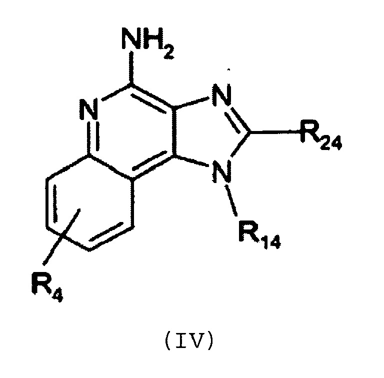 dans laquelle
R₁₄ est -CHR_{A}R_{B} dans lequel R_{B} est un atome d'hydrogène ou une liaison carbone-carbone, à condition que lorsque R_{B} est un atome d'hydrogène, R_{A} soit un groupe alcoxy ayant de un à environ quatre atomes de carbone, un groupe hydroxyalcoxy ayant de un à environ quatre atomes de carbone, un groupe 1-alcynyle ayant de deux à environ dix atomes de carbone, un groupe tétrahydropyranyle, un groupe alcoxyalkyle dans lequel la fraction alcoxy contient de un à environ quatre atomes de carbone et la fraction alkyle contient de un à environ quatre atomes de carbone, un groupe 2-, 3-, ou 4-pyridyle, et à la condition supplémentaire que lorsque R_{B} est une liaison carbone-carbone, R_{B} et R_{A} ensemble forment un groupe tétrahydrofuranyle facultativement substitué par un ou plusieurs substituants indépendamment choisis dans le groupe constitué par un groupe hydroxy et hydroxyalkyle ayant de un à environ quatre atomes de carbone ; R₂₄ est choisi dans le groupe constitué par un atome d'hydrogène, un groupe alkyle ayant de un à environ quatre atomes de carbone, un groupe phényle, et un groupe phényl substitué dans lequel le substituant est choisi dans le groupe constitué par un groupe alkyle ayant de un à environ quatre atomes de carbone, un groupe alcoxy ayant de un à environ quatre atomes de carbone, et un atome d'halogène ; et R₄ est choisi dans le groupe constitué par un atome d'hydrogène, un groupe alcoxy à chaîne linéaire ou ramifiée contenant de un à environ quatre atomes de carbone, un atome d'halogène, et un groupe alkyle à chaîne linéaire ou ramifiée contenant de un à environ quatre atomes de carbone ; 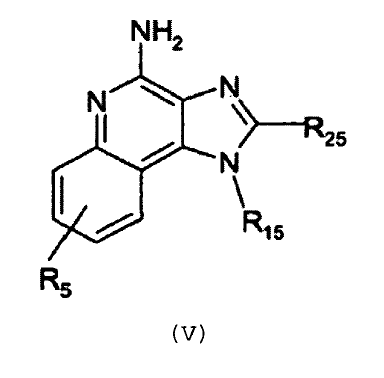 dans laquelle
R₁₅ est choisi dans le groupe constitué par : un atome d'hydrogène ; un groupe alkyle à chaîne linéaire ou ramifiée contenant de un à environ dix atomes de carbone et un groupe alkyle à chaîne linéaire ou ramifiée, substitué, et contenant de un à environ dix atomes de carbone, le substituant étant choisi dans le groupe constitué par un groupe cycloalkyle contenant de trois à environ six atomes de carbone et un groupe cycloalkyle contenant de trois à environ six atomes de carbone substitués par un groupe alkyle à chaîne linéaire ou à chaîne ramifiée contenant de un à environ quatre atomes de carbone ; un groupe alcényle à chaîne linéaire ou ramifiée contenant de deux à environ dix atomes de carbone et un groupe alcényle à chaîne linéaire ou ramifiée, substitué, et contenant de deux à environ dix atomes de carbone, le substituant étant choisi dans le groupe constitué par un groupe cycloalkyle contenant de trois à environ six atomes de carbone et un groupe cycloalkyle contenant de trois à environ six atomes de carbone substitués par un groupe alkyle à chaîne linéaire ou à chaîne ramifiée contenant de un à environ quatre atomes de carbone ; un groupe hydroxyalkyle ayant de un à environ six atomes de carbone ; un groupe alcoxyalkyle dans lequel la fraction alcoxy contient de un à environ quatre atomes de carbone et la fraction alkyle contient de un à environ six atomes de carbone ; un groupe acyloxyalkyle dans lequel la fraction acyloxy est un groupe alcanoyloxy de deux à environ quatre atomes de carbone ou un groupe benzoyloxy, et la fraction alkyle contient de un à environ six atomes de carbone ; un groupe benzyle ; (phényl)éthyle ; et phényle ; ledit substituant benzyle, (phényl)éthyle ou phényle étant facultativement substitué sur le cycle benzène par une ou deux fractions indépendamment choisies dans le groupe constitué par un groupe alkyle ayant de un à environ quatre atomes de carbone, un groupe alcoxy ayant de un à environ quatre atomes de carbone, et un atome d'halogène, à condition que lorsque ledit cycle benzène est substitué par deux desdites fractions, alors les fractions ensemble ne contiennent pas plus de six atomes de carbone ;
R₂₅ est 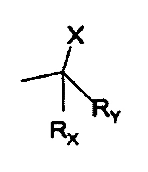 dans laquelle
R_{X} et R_{Y} sont indépendamment choisis dans le groupe constitué par un atome d'hydrogène, un groupe alkyle ayant de un à environ quatre atomes de carbone, un groupe phényle, et un groupe phényl substitué dans lequel le substituant est choisi dans le groupe constitué par un groupe alkyle ayant de un à environ quatre atomes de carbone, un groupe alcoxy ayant de un à environ quatre atomes de carbone, et un atome d'halogène ; X est choisi dans le groupe constitué par un groupe alcoxy contenant de un à environ quatre atomes de carbone, un groupe alcoxyalkyle dans lequel la fraction alcoxy contient de un à environ quatre atomes de carbone et la fraction alkyle contient de un à environ quatre atomes de carbone, un groupe haloalkyle ayant de un à environ quatre atomes de carbone, un group alkylamido dans lequel le groupe alkyle contient de un à environ quatre atomes de carbone, un groupe amino, un groupe amino substitué dans lequel le substituant est un groupe alkyle ou hydroxyalkyle contenant de un à environ quatre atomes de carbone, un groupe azido, un groupe alkylthio contenant de un à environ quatre atomes de carbone ; et R₅ est choisi dans le groupe constitué par un atome d'hydrogène, un groupe alcoxy à chaîne linéaire ou ramifiée contenant de un à environ quatre atomes de carbone, un atome d'halogène, et un groupe alkyle à chaîne linéaire ou ramifiée contenant de un à environ quatre atomes de carbone ; 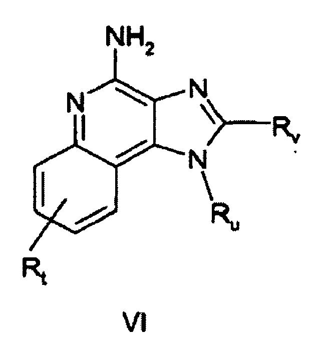 dans laquelle
Rₜ est choisi dans le groupe constitué par un atome d'hydrogène, un groupe alcoxy à chaîne linéaire ou ramifiée contenant de un à environ quatre atomes de carbone, un atome d'halogène, et un groupe alkyle à chaîne linéaire ou ramifiée contenant de un à environ quatre atomes de carbone ;
Rᵤ est un groupe 2-méthylpropyle ou 2-hydroxy-2-méthylpropyle ; et
Rᵥ est un atome d'hydrogène, un groupe alkyle ayant de un à environ six atomes de carbone, ou un groupe alcoxyalkyle dans lequel la fraction alcoxy contient de un à environ quatre atomes de carbone et la fraction alkyle contient de un à environ quatre atomes de carbone,
ou un sel pharmaceutiquement acceptable de l'un quelconque de ce qui précède.

4. Vaccin selon la revendication 3, dans lequel le dérivé de 1H-imidazo[4,5-c]quinolin-4-amine est un composé de formule VI.

5. Vaccin selon la revendication 4, dans lequel Rₜ est un atome d'hydrogène.

6. Vaccin selon la revendication 5, dans lequel Rᵤ est un groupe 2-méthylpropyle ou 2-hydroxy-2-méthylpropyle, et Rᵥ est un atome d'hydrogène, un groupe méthyle, ou éthoxyméthyle.

7. Vaccin selon la revendication 6, dans lequel le composé est choisi dans le groupe constitué par :
la 1-(2-méthylpropyl)-1H-imidazo[4,5-c]quinolin-4-amine
la 1-(2-hydroxy-2-méthylpropyl)-1H-imidazo[4,5-c]quinolin-4-amine
la 1-(2-hydroxy-2-méthylpropyl)-2-méthyl-1H-imidazo[4,5-c]quinolin-4-amine
la 1-(2-hydroxy-2-méthylpropyl)-2-éthoxyméthyle-1H-imidazo[4,5-c]quinolin-4-amine.

8. Vaccin selon l'une quelconque des revendications précédentes, dans lequel chaque composant est sous une forme adaptée pour une administration via l'une quelconque des voies orale, nasale, topique, pulmonaire, intramusculaire, sous-cutanée ou intradermique.

9. Vaccin selon la revendication 8, dans lequel le composant immunogène se trouve sous une forme adaptée pour une administration utilisant une technique de transfert de gènes médié par des particules.

10. Vaccin selon la revendication 9, dans lequel le composant adjuvant se trouve sous une forme adaptée pour une administration utilisant une technique de transfert de gènes médié par des particules.

11. Utilisation d'un dérivé de 1H-imidazo[4,5-c]quinolin-4-amine dans la fabrication d'un médicament destiné à améliorer les réponses immunitaires initiées par un peptide antigénique dans le traitement d'un mammifère dans un état pathologique choisi parmi une infection virale, bactérienne ou parasitaire, un cancer, une allergie ou un trouble auto-immun, ledit peptide étant exprimé en résultat de l'administration à un mammifère d'une séquence de nucléotides codant pour ledit peptide, et le dérivé de 1H-imidazo[4,5-c]quinolin-4-amine est administré entre environ 1 jour avant et environ 3 jours après l'administration de la séquence de nucléotides codant pour ledit peptide.

12. Utilisation selon la revendication 11, dans laquelle le dérivé de 1H-imidazo[4,5-c]quinolin-4-amine est tel que défini dans la revendication 3.

13. Utilisation selon la revendication 12, dans laquelle le dérivé de 1H-imidazo[4,5-c]quinolin-4-amine est tel que défini dans la revendication 4.

14. Utilisation selon l'une quelconque des revendications 11 à 13, dans laquelle le dérivé de 1H-imidazo[4,5c]quinolin-4-amine est administré en une dose comprise entre environ 1 mg/kg et 50 mg/kg.

15. Utilisation selon l'une quelconque des revendications 11 à 14, dans laquelle la séquence de nucléotides codant pour ledit peptide est administrée par une technique de transfert de gènes médié par des particules et le dérivé de 1H-imidazo[4,5-c]quinolin-4-amine est administré par voie topique au site d'administration de la séquence de nucléotides.

16. Dérivé de 1H-imidazo[4,5-c]quinolin-4-amine destiné à être utilisé en tant qu'un adjuvant pour améliorer une réponse immunitaire initiée par le peptide antigénique, ledit peptide étant exprimé en résultat de l'administration à un mammifère d'une séquence de nucléotides codant pour ledit peptide, le dérivé de 1H-imidazo[4,5-c]quinolin-4-amine étant administré entre environ 1 jour avant et environ 3 jours après l'administration de la séquence de nucléotides codant pour ledit peptide.

17. Dérivé de 1H-imidazo[4,5-c]quinolin-4-amine selon la revendication 16, dans lequel le dérivé de 1H-imidazo[4,5-c]quinolin-4-amine est tel que défini dans la revendication 3.

18. Dérivé de 1H-imidazo[4,5-c]quinolin-4-amine selon la revendication 17, dans lequel le dérivé de 1H-imidazo[4,5-c]quinolin-4-amine est tel que défini dans la revendication 4.
